(19) Europäisches Patentamt
European Patent Office
Office européen des brevets

(11) Numéro de publication: **0 576 619 B1**

# (12) FASCICULE DE BREVET EUROPEEN

(45) Date de publication de fascicule du brevet: **25.01.95**

(51) Int. Cl.[6]: **C07D 339/04**, C07D 409/06, C07D 515/04, A61K 31/385

(21) Numéro de dépôt: **92910888.4**

(22) Date de dépôt: **30.04.92**

(86) Numéro de dépôt internationale :
**PCT/FR92/00392**

(87) Numéro de publication internationale :
**WO 92/19613 (12.11.92 92/28)**

## (54) COMPOSITIONS THERAPEUTIOUES A BASE DE DERIVES DE 1,2-DITHIOLE-3-THIONE.

(30) Priorité: **02.05.91 FR 9105411**

(43) Date de publication de la demande:
**05.01.94 Bulletin 94/01**

(45) Mention de la délivrance du brevet:
**25.01.95 Bulletin 95/04**

(84) Etats contractants désignés:
**BE CH DE ES FR GB IT LI NL**

(56) Documents cités:
**US-A- 4 190 727**

**Chemical Abstracts, vol. 113, 1990, (Columbus Ohio, US), B.A. TEICHER et al.: "1,2-Dithiol-3-thione and dithioester analogs: potentioal radioprotectors", see page 348, abstract No. 207595v, & BR. J: CANCER 1990, 62(1), 17-22, see abstract**

(73) Titulaire: **LABORATORIES DE THERAPEUTIOUE MODERNE**
**42, Rue Rouget-de-Lisle**
**F-92161 SuresnesCédex (FR)**

(72) Inventeur: **CHRISTEN, Marie-Odile**
**27, avenue Marceau**
**F-75116 Paris (FR)**
Inventeur: **BURGOT, Jean-Louis**
**207, route de Fougères**
**F-35700 Rennes (FR)**

(74) Mandataire: **Le Guen, Gérard et al**
**CABINET LAVOIX**
**2, place d'Estienne d'Orves**
**F-75441 Paris Cédex 09 (FR)**

EP 0 576 619 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Chemical Abstracts, vol. 114, 1991, (Columbus, Ohio, US), H.D. STACHEL et al.: "Ogston's color test", see page 762, column 2, abstract No. 164073z, & ARCH. PHARM: (WEINHEIM, GER.) 1991, 324(2), 131-2, see abstract (cited in the application)

Chemical Abstracts, vol. 67, 1967, (Columbus, Ohio, US), J. FAUST et al.: "Organic sulfur compunds, LXXXVI, 1,2-Dithiol-3-one-5-carboxylic acid chloride and its derivatives", see page 7745, column 1, abstract No. 82141s, & Z. CHEM. 7(7), 275-6(1967), see abstract (cited in the application)

Chemical Abstracts, vol. 66, 1967, (Columbus, Ohio, US), J. FABIAN et al.: "Infrared absorptions of 3H-1,2-dithiol-3-thiones (tithiones)", see pabe 2269, column 2, abstract No. 23919h, & CHEM. InD: (LONDON) 1966(47), 1962-3, see abstract

Bulletin de la Société Chimique de France, vol. 2, No. 2, 1973, (Paris, FR), C. TREBAUL: XI. SYnthèse de quelques dérives carbonylés en 5 de la phényl-4 dithiole-1,2 one-*", pages 721-723, see pages 721-723 (cited in the application)

## Description

La présente invention concerne des compositions thérapeutiques à base de dérivés de 1,2-dithiole-3-thione.

Comme état de la technique, on peut mentionner l'anéthole trithione ou 5-(p-méthoxyphényl)-3H-1,2-dithiole-3-thioneutilisée en thérapeutique comme cholérétique (US-A-2 556 963) et des composés de formule :

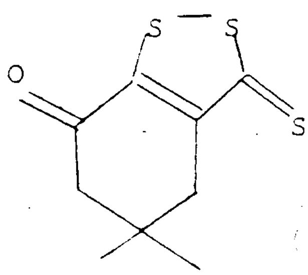

( A )

et

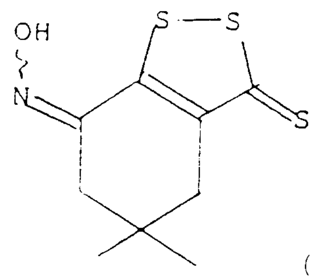

( B )

décrits par Ebel et al, Bull. Soc. Chim. Fr. 1963, 161, le composé A étant obtenu par réaction du soufre sur l'isophorone et le composé B étant obtenu à partir du composé A par réaction avec $NH_2OH$.

Teicher et al. (Br. J. Cancer, 1990, 62 17) décrivent l'utilisation de 1,2-dithiole-3-thiones et en particulier de 5-(2-thiényl)-1,2-dithiole-3-thione, comme radioprotecteurs.

La présente invention a pour objet des compositions thérapeutiques contenant, à titre de principe actif, un composé choisi parmi des composés de formules:

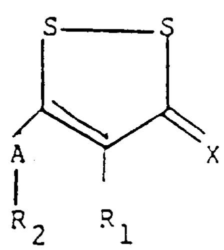

(I)

et

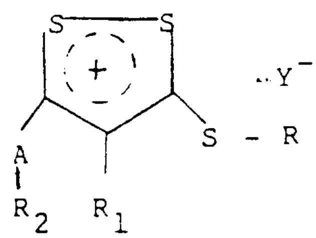

(Ia)

dans lesquelles :

- X est choisi parmi =S, =O, =N-OH, =N-$R_5$, $R_5$ étant un groupe alkyle en $C_1$-$C_6$ ou aryle, =N-NH-CO-$NH_2$ et =N-NH-CS-$NH_2$, et

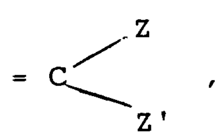

  Z et Z' étant des groupes attracteurs d'électrons tels que des groupes ester ou cyano,
- A est choisi parmi le groupe >C=N-OH, un groupe de formule >C=N-$OR_3$
  (où $R_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$, un groupe aryl(alkyle en $C_1$-$C_6$), un groupe (alkyl en $C_1$-$C_6$) carbonyle et un groupe aryl(alkyl en $C_1$-$C_6$) carbonyle),
  un groupe >C=O, un groupe >C=N-$R_4$, $R_4$ étant un groupe alkyle en $C_1$-$C_6$ ou un groupe aryle, et un groupe CHOH,
- $R_1$ et $R_2$ sont choisis indépendamment l'un de l'autre parmi l'hydrogène, un halogène, un groupe nitro, un groupe nitroso, un groupe thiocyano, un groupe alkyle en $C_1$-$C_6$, un groupe alcényle en $C_2$-$C_6$, un groupe aryle, un groupe aryl(alkyle en $C_1$-$C_6$), un groupe aryl(alcényle en $C_2$-$C_6$), un groupe carboxy, un groupe (alkyl en $C_1$-$C_6$) carbonyle, un groupe arylcarbonyle, un groupe (alcoxy en $C_1$-$C_6$)

carbonyle, un groupe (alkoxy en $C_1$-$C_6$) carbonyl(alkyle en $C_1$-$C_6$), un groupe alcoxy en $C_1$-$C_6$, un groupe trifluorométhyle, un groupe amino, un groupe di(alkyl en $C_1$-$C_6$) amino(alkyl en $C_1$-$C_6$), un groupe acylamino de formule $-NHCOC_nH_{2n+1}$ avec n de 0 à 6, un groupe $-NH-CSC_nH_{2n+1}$ avec n de 0 à 6, un groupe terpényle, un groupe cyano, un groupe alcynyle en $C_2$-$C_6$, un groupe alcynyle en $C_2$-$C_6$ substitué par un groupe alkyle en $C_1$-$C_6$ ou aryle, un groupe hydroxy(alkyle en $C_1$-$C_6$) (acyl en $C_1$-$C_6$)oxy(alkyle en $C_1$-$C_6$), un groupe (alkyl en $C_1$-$C_6$)thio, un groupe arylthio,
ou bien $R_1$ et $R_2$ forment ensemble un groupe alkylène mono ou polycyclique en $C_2$-$C_{20}$ comprenant éventuellement un ou plusieurs hétéroatomes, à l'exception du groupe 2,2-diméthyltriméthylène, ou un groupe cycloalkylène en $C_3$-$C_{12}$,
- R est choisi parmi un groupe alkyle en $C_1$-$C_6$,
- $Y^-$ est un anion pharmaceutiquement acceptable, tel que halogénure ou sulfate,
et leurs sels pharmaceutiquement acceptables.

L'invention a en particulier pour objet une composition pharmaceutique comprenant, à titre d'ingrédient actif, un composé choisi parmi les composés de formules :

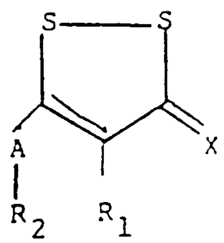 (I) et 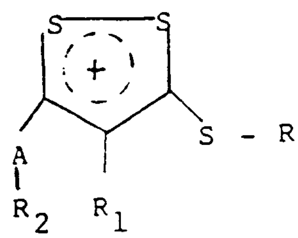 (Ia)

dans lesquelles :
- X est choisi parmi S et O,
- A est choisi parmi le groupe $>C=N-OH$, un groupe de formule $>C=N-OR_3$
(où $R_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$, un groupe aryl(alkyle en $C_1$-$C_6$), un groupe (alkyl en $C_1$-$C_6$) carbonyle et un groupe aryl(alkyl en $C_1$-$C_6$) carbonyle),
un groupe $C=O$, un groupe $>C=N-R_4$, $R_4$ étant un groupe alkyle en $C_1$-$C_6$ ou un groupe aryle, et un groupe CHOH, - $R_1$ et $R_2$ sont choisis indépendamment l'un de l'autre parmi l'hydrogène, un halogène, un groupe alkyle en $C_1$-$C_6$,un groupe aryle, un groupe aryl(alkyle en $C_1$-$C_6$), un groupe carboxy, un groupe alcoxycarbonyle, et un groupe alcoxy en $C_1$-$C_6$, un groupe trifluorométhyle, un groupe di(alkyl en $C_1$-$C_6$) amino(alkyl en $C_1$-$C_6$) et un groupe acylamino de formule $-NHCOC_nH_{2n+1}$ avec n de 0 à 6, ou bien $R_1$ et $R_2$ forment ensemble un groupe alkylène en $C_2$-$C_{12}$ , à l'exception du groupe 2,2-diméthyltriméthylène, ou un groupe cycloalkylène en $C_3$-$C_{12}$,
- R est choisi parmi un groupe alkyle en $C_1$-$C_6$,
et leurs sels pharmaceutiquement acceptables.

Dans la définition qui précède, par groupe aryle ou fraction aryle d'un groupe arylalkyle, on désigne un groupe carboné aromatique tel qu'un groupe phényle ou naphtyle ou un groupe hétérocyclique aromatique tel qu'un groupe thiényle ou furyle, groupes qui peuvent porter un ou plusieurs substituants choisis parmi un atome d'halogène, un groupe alkyle en $C_1$-$C_4$, un groupe alcoxy en $C_1$-$C_4$, un groupe trifluorométhyle, un groupe nitro et un groupe hydroxy.

Parmi les composés de formule I, certains sont connus.

C'est ainsi que Stachel et al. (Arch. Pharm. 1991, 324, 2, 131) décrivent des 1,2-dithiole-3-thiones de formule I dans laquelle A = CO, X = S, $R_1$ = $OCH_3$ et $R_2$ = $OCH_3$ ou $NH_2$.

Faust et al. (Z Chem., 1967, 7, 7, 275) décrivent des composés de formule I dans laquelle A = CO, $R_1$ = H, X = S et $R_2$ = Cl, $OC_2H_5$, $NH_2$ ou X = O et $R_2$ = Cl, $OC_2H_5$, $NH_2$, $NHC_6H_5$.

De plus, J. Fabian et ai. (Chem. Ind., 1966, 1962-3) décrivent une 1,2-dithiole-3-thione de formule I dans laquelle X = S, $AR_2$ = $CO-OC_2H_5$ et $R_1$ = H.

Trebaul (Bul. Soc. Chim., 1973, 2, 2, 721) décrit des composés de formule I dans laquelle A = CO $R_1$ = $C_6H_5$, X = S et $R_2$ = $OC_2H_5$, $NH_2$, $N(C_6H_5)_2$ ou X = O et $R_2$ = $OC_2H_5$, Cl, $pOCH_3C_6H_4$, $NH_2$, $NHC_6H_5$, $N(C_6H_5)_2$.

Par ailleurs, quelques composés de formule I dans laquelle A = =N-Ar et X = S sont décrits par Quiniou (Bul. Soc. Chem., 1960, 5, 47) et dans US-A-4 190 727.

Un premier groupe de composés de formule I est formé par ceux dans lesquels A est un groupe $>C=N\text{-}OH$, c'est-à-dire des oximes de formule

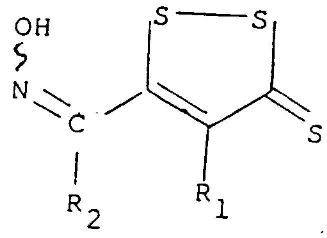

(II)

Ces composés, qui sont nouveaux, peuvent être préparés selon l'invention à partir de 1,2-dithiole-3-thiones de formule :

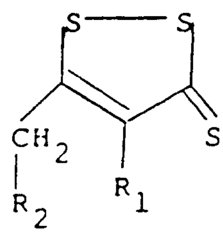

(III)

par action de l'acide nitreux ou du nitrite d'isoamyle.

En pratique, on peut faire réagir sur un composé de formule III du nitrite de sodium en milieu acide acétique glacial à environ 40° C ou du nitrite d'isoamyle en présence d'éthylate de sodium en milieu éthanol à 0° C ou à température ambiante.

Un certain nombre de composés de formule III sont connus. Les autres peuvent être préparés selon des procédés connus (voir notamment Thuillier A., Vialle J., Bull. Soc. Chim. Fr., 1962, 2187, Legrand L., Lozach N., Bull. Soc. Chim. Fr., 1955, 79).

Il est à noter que la réaction de nitrite de sodium en milieu acide acétique glacial sur les composés de formule III conduit généralement à la formation non seulement des oximes de formule II mais également à la formation de composés de formule :

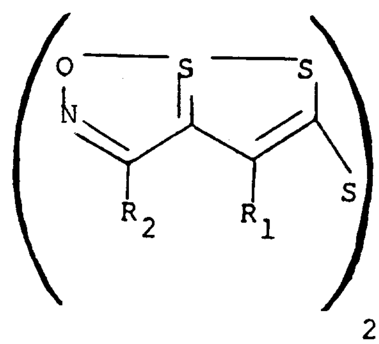

(V)

qui constituent des produits intermédiaires et qui conduisent aux oximes de formule II par action de la soude, ainsi qu'éventuellement des composés de formule

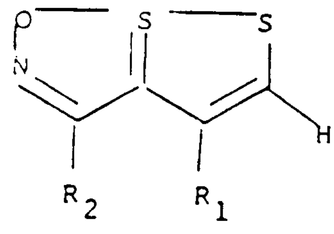

(IV)

qui constituent des produits secondaires.

Il est à noter également que la réaction du nitrite d'isoamyle sur les composés de formule III ne conduit qu'à la formation d'oximes de formule II.

Un second groupe de composés de formule I est formé par ceux dans lesquels A est un groupe $>C=O$, c'est-à-dire des aldéhydes ou cétones de formule

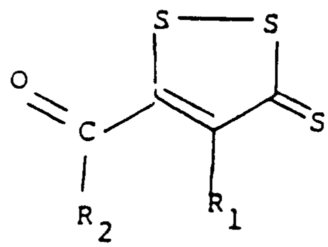

VI

Ces composés de formule VI peuvent être préparés par réaction du formaldéhyde en milieu acide sur une oxime de formule II.

Parmi les composés de formule VI, certains sont nouveaux. C'est le cas en particulier des composés VIa répondant à la formule VI mais dans laquelle $R_1$ est un groupe alkykle ou arylalkyle ou un atome d'halogène, des composés VIb répondant à la formule VI mais dans laquelle $R_1$ est un groupe aryle et $R_2$ est un atome d'hydrogène ou un groupe alkyle et des composés VIc répondent à la formule VI mais dans laquelle $R_1$ est un atome d'hydrogène et $R_2$ est un groupe alkyle.

Un troisième groupe de composés de formule I est formé par ceux dans lesquels A est un groupe

$C=N\text{-}OR'_3$ où $R'_3$ est un groupe alkyle en $C_1$-$C_6$, éventuellement substitué, notamment par un ou plusieurs groupes choisis parmi les groupes hydroxy, amino, chloro et alkoxy en $C_1$-$C_4$ ou un groupe aryl-(alkyle en $C_1$-$C_6$), c'est-à-dire des composés de formule

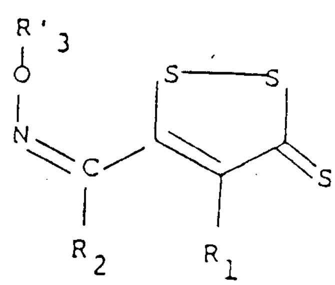

VII

Ces composés peuvent être obtenus par alkylation d'une oxime de formule II à l'aide d'un halogénure $R'_3$-Hal en présence d'éthylate de sodium.

On a par ailleurs découvert que si on effectue une alkylation d'une oxime de formule II avec un halogénure $R'_3$-Hal mais en présence d'une solution aqueuse d'hydroxyde de sodium, on obtient non pas des composés de formule VII mais des composés de formule

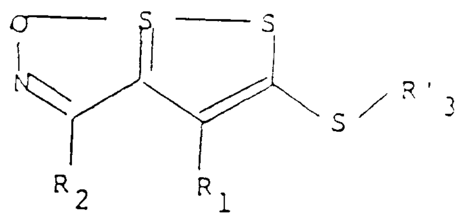

VIII

dans laquelle R'3 a la signification donnée ci-dessus.

Il est possible d'expliquer cette obtention par le fait que les oximes de formule II possèdent une forme tautomèrethiol :

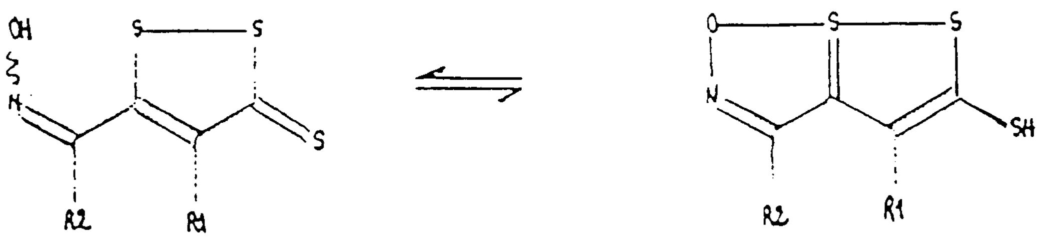

Un quatrième groupe de composés de formule I est formé par les composés dans lesquels A est un groupe C=N-O-CO-R"$_3$, R"$_3$ étant choisi parmi un atome d'hydrogène, un groupe alkyle en $C_1$-C6 éventuellement substitué, un groupe aryle et un groupe aryl(alkyle en $C_1$-$C_6$), c'est-à-dire des composés de formule

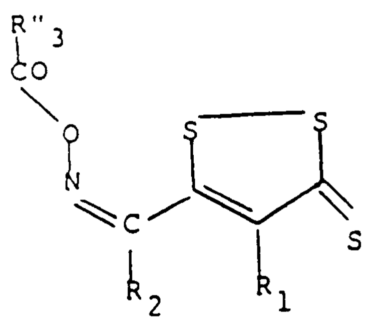

IX

dans laquelle R"$_3$ a la signification donnée précédemment.

Ces composés peuvent être obtenus par acylation d'une oxime de formule II par un chlorure d'acide R"$_3$COCl en milieu toluénique en présence de pyridine.

Un cinquième groupe de composés de formule I est formé par les composés dans lesquels A est un groupe CH-OH, c'est-à-dire les composés de formule

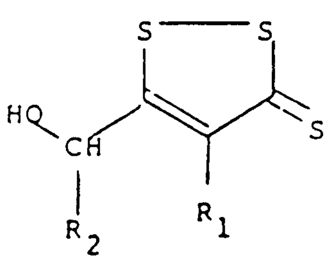

X

Ces composés peuvent généralement être obtenus par réduction d'un composé de formule VI.

Un sixième groupe de composés de formule I est formé par les composés dans lesquels A est un groupe C=N-$R_4$, $R_4$ étant un groupe alkyle en $C_1$-$C_6$ ou aryle, c'est-à-dire des composés de formule

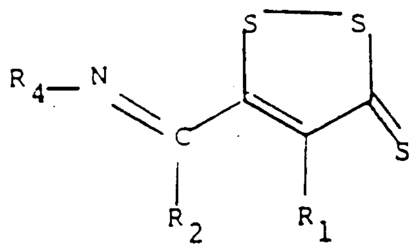

XI

Ces composés peuvent être obtenus à partir des composés de formule VI par action d'une amine de formule $R_4$-$NH_2$.

Un septième groupe de composés de formule I est formé par les composés dans lesquels A est un groupe C = O et X est un atome d'oxygène, c'est-à-dire des composés de formule :

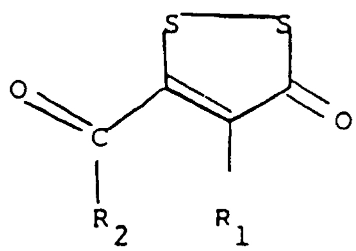

(XII)

dans laquelle $R_1$ et $R_2$ ont la signification donnée ci-dessus. Ces composés peuvent être obtenus à partir des composés de formule VI par action de l'oxyde de benzonitrile.

Plus généralement, les composés de formule I dans laquelle X = O peuvent être obtenus à partir des composés de formule I dans laquelle X = S par action de l'oxyde de benzonitrile.

Les oximes de formule VI peuvent en outre réagir à froid avec l'acétylène dicarboxylate de méthyle en solution acétonique selon Davy et Decrouen (Bull. Soc. Chim., 1976, 115) pour donner des composés de formule

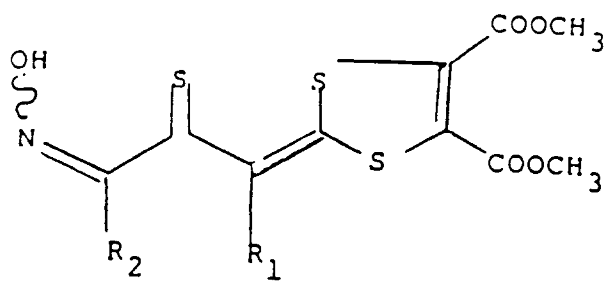

(XIII)

Les composés de formule Ia peuvent être obtenus à partir des composés de formule I par réaction avec un iodure d'alkyle de formule IR, R étant un groupe alkyle en $C_1$-$C_6$.

Les exemples suivants illustrent la présente invention.

**I - Préparation des oximes de formule II, des disulfures de formule V, des oxadithiazapentalènes de formule IV**

1 - Utilisation de nitrite de sodium en milieu acide acétique glacial.

Exemple

Dans un erlenmeyer de 250 ml muni d'une agitation magnétique, on dissout 1 g de 4,5-diméthyl-1,2-dithiole-3-thione dans 30 ml d'acide acétique glacial porté à ébullition. On laisse refroidir à 40° C. On ajoute par petites portions 2 g de nitrite de sodium à l'état solide. Il se forme immédiatement un précipité

ocre. Après 30 mn, on étend la solution acétique avec de l'eau. On filtre le précipité abondant qui est constitué essentiellement par le disulfure de formule V et des traces de la 1,2- dithiole-3-thione de départ. Le précipité est ajouté à une solution d'hydroxyde de sodium à 20 %. Après agitation à 60° C durant 2 heures, il y a dissolution presque complète avec changement de coloration. La solution aqueuse devient marron foncé. Elle est extraite par du toluène puis est acidifiée sous refroidissement. Il y a apparition d'un précipité de l'oxime brute de coloration jaune orangé. Le reste d'oxime qui n'a pas précipité est extrait soit par une solution d'éther sulfurique soit par une solution de dichlorométhane contenant 5 % d'acétone.

D'autre part, on extrait la solution acétique obtenue après filtration de l'oxime brute par une solution toluénique qui est regroupée avec celle ayant servi à l'extraction de la phase sodée. La solution toluénique est lavée à l'eau, séchée sur sulfate de sodium, concentrée et chromatographiée sur silicagel. L'oxadithiazapentalène est élué après le dithiolethione de départ avec du toluène.

La structure des oximes de formule II est déterminée d'après l'étude des spectres de RMN$^1$H, 13C, I.R., masse et d'après les microanalyses. L'attribution des structures syn et anti pour l'oxime dont la préparation est décrite ci-dessus est effectuée d'après les valeurs des constantes de couplage $J^1$(C-H) dans les enchaînements ci-dessous :

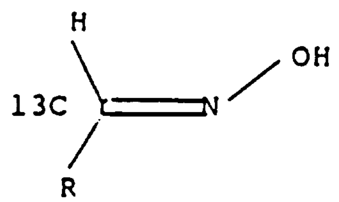

**isomère syn**

**$J^1$(C-H) = 175,4 Hz**

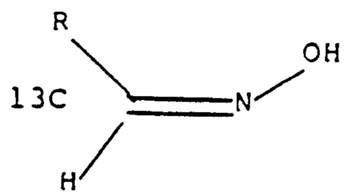

**isomère anti**

**$J^1$(C-H) = 185,7 Hz**

On a rassemblé dans les tableaux I et II les caractéristiques des produits obtenus ainsi que celles d'autres produits de formules II, IV et V.

TABLEAU I

| R1 | R2 | Oximes de formule II | | | Oxadithiazapentalènes de formule IV | | |
|---|---|---|---|---|---|---|---|
| | | Code | Rdt% | F°C | Code | Rdt% | F°C |
| $CH_3$ | H | 1 | 65syn(M*) + anti | 174 | 21 | 30 | 117 |
| $CH_3$ | CH3 | 2 | 50syn(M*) + anti | 138 | 22 | 40 | 120 |
| H | H | 2 | 20 | 143 | 23 | 10 | 83 |
| H | $C_6H_5$ | 4 | 30 | 165 | 24 | 15 | 118 |
| $OCH_3$ | H | 5 | 30 | 108 | | | |
| H | | 6 | 20 | 142 | | | |
| | H | 7 | 15 | 139 | | | |
| H | $CH_3$ | 8 | 55 | 181 | 25 | 30 | 93 |
| $C_2H_5$ | $CH_3$ | 9 | 50 | 92 | 26 | 20 | 90 |
| $CH_3$ | $CH_3CHCH_3$ | 10 | 45 | 113 | 27 | 30 | 160 |

(*) M : majoritaire

TABLEAU II

| R1 | R2 | Oximes de formule II | | | Dimères de formule V | | |
|---|---|---|---|---|---|---|---|
| | | Code | Rdt% | F ° C | Code | Rdt% | F ° C |
| $-(CH_2)_3$ | | 11 | 50 | 195 | 31 | 25 | 120-21 |
| $C_6H_5$ | $CH_3$ | 12 | 55 | 163 | 32 | 25 | 181-82 |
| $C_6H_5$ | $C_6H_5$ | 13 | 20 | 171 | 33 | 30 | 190-91 |
| $COOC_2H_5$ | $C_6H_5$ | 14 | 40 | 194 | 34 | 25 | 163-64 |
| $CH_2C_6H_5$ | H | 15 | 80 | 143 | 35 | 15 | 106-8 |
| $CH_2C_6H_5$ | $CH_3$ | 16 | 50 | 122 | | | |
| H | $C_2H_5$ | 17 | 55 | 130 | 36 | 30 | 143-155 |
| $C_6H5$ | $CH_2C_6H_5$ | 18 | 40 | 157 | 37 | 20 | 102-103 |
| $C_2H_5$ | H | 19 | 50 | 149-160 | | | |
| Cl | H | 20 | 45 | 230 | | | |

2 - Utilisation du nitrite d'isoamyle.

Exemple

A une solution éthanolique contenant deux équivalents d'éthylate de sodium, on ajoute en 1/2 heure une solution éthanolique de la 5-méthyl-1,2- dithiole-3-thione (1 équivalent) avec la quantité équivalente de nitrite d'isoamyle à température ambiante. On laisse sous agitation une nuit. On jette dans l'eau et extrait l'oxime (composé 3) par une solution d'éther éthylique. La solution éthérée est lavée à l'eau, séchée sur sulfate de sodium, concentrée et purifiée par chromatographie sur silicagel. L'oxime est éluée après la dithiolethione de départ dont on récupère environ 40 % de la quantité initiale.

**II - Préparation des composés de formule VI.**

Exemple

On met en présence 2 g de dithiolethione oxime de formule II, 25 ml d'une solution aqueuse de formaldéhyde à 37 % acidifée par quelques gouttes d'acide chlorhydrique concentré, 100 ml de toluène. On porte à reflux en agitant pendant 2 heures. La solution toluénique devient rouge foncé très vite.

Après refroidissement la phase toluénique est décantée, lavée à l'eau jusqu'à neutralité, séchée sur sulfate de sodium, concentrée et chromatagraphiée sur silicagel. Les dithiolethione aldéhydes ou cétones de formule VI sont elués au mélange éther de pétrole/ toluène 60/40.

Leurs structures sont établies à partir des procédés classiques de l'analyse structurale y compris à partir de l'analyse élémentaire. En particulier, en spectroscopie I.R., ces composés présentent tous une bande $\nu_{C=O}$ . Leurs caractéristiques sont données dans le tableau III.

TABLEAU III

| Code | R1 | R2 | F°C | $\nu_{C=O}$ ($cm^{-1}$) |
|---|---|---|---|---|
| 41 | $CH_3$ | H | 105 | 1660 |
| 42 | $C_6H_5$ | H | 132 | 1625 |
| 43 | $-CH_2-CH_2-CH_2-$ | | 95 | 1670 |
| 44 | $CH_3$ | $CH_3$ | 76 | 1690 |
| 45 | H | H | 100 | 1670 |
| 46 | $C_6H_5$ | $CH_3$ | 86 | 1670 |
| 47 | $OCH_3$ | H | 101 | 1660 |
| 48 | H | $CH_3$ | 115 | 1660 |
| 49 | $CH_2C_6H_5$ | H | 124 | 1665 |
| 50 | $C_2H_5$ | $CH_3$ | 125 | 1670 |
| 51 | $CH_3$ | $CH_3-CH-CH_3$ | 59 | 1690 |
| 52 | $CH_2C_6H_5$ | $CH_3$ | 63 | 1670 |
| 53 | H | $CH_2-CH_3$ | 116 | 1680 |
| 54 | $C_2H_5$ | H | 75-76 | 1675 |
| 55 | Cl | H | 136 | 1680 |

**III - Préparation des composés de formule VII.**

A une solution éthanolique contenant 2 équivalents d'éthylate de sodium, on ajoute en 1/2 heure 1 équivalent de l'oxime II 1 en solution éthanolique, en refroidissant extérieurement par un bain d'eau glacée. On additionne alors 1,1 équivalent d'iodure de méthyle. On laisse agir à température ambiante une quinzaine d'heures. On étend alors la solution par de l'eau, extrait au toluène, sèche sur sulfate de soude et concentre la solution toluénique.

Le résidu est chromatographié sur silicagel.

La 5-méthoxyiminométhyl 4-méthyl 1,2-dithiole 3-thione (VII 1) est éluée au mélange éther de pétrole/toluène (90/10).

C'est un solide rouge F = 93-94° C.

RMN$^1$H (300 MHz) ($CDCl_3$) $\delta$ ppm/TMS = H (s:8,30); $OCH_3$ (s:4,10); $CH_3$ (s:2,30).

**IV - Préparation des composés de formule VIII.**

On dissout l'oxime II dans une solution aqueuse d'hydroxyde de sodium à 10 %; on ajoute un excès d'iodure de méthyle à froid et on laisse agiter à température ambiante pendant une nuit.

On extrait alors avec une solution toluénique qui est lavée à l'eau, séchée, concentrée et chormatographiée sur silicagel.

Les 5-(méthylthiooxadithiazapentalènes) VIII sont élués au mélange éther de pétole/toluène (60/40).

On a rassemblé dans le tableau IV les caractéristiques de composés de formule VIII.

TABLEAU IV

| Code | $R_1$ | $R_2$ | $R'_3$ | F°C | δppm/$CDCl_3$ méthylthio |
|---|---|---|---|---|---|
| 61 | $CH_3$ | H | $CH_3$ | 115 | 2,79 |
| 62 | $C_6H_5$ | H | $CH_3$ | 118 | 2,70 |
| 63 | | $-CH_2-CH_2-CH_2-$ | $CH_3$ | 114 | 2,75 |
| 64 | $CH_3$ | $CH_3$ | $CH_3$ | 107 | 2,70 |
| 65 | H | H | $CH_3$ | 106 | 2,79 |
| 66 | $C_6H_5$ | $CH_3$ | $CH_3$ | 160 | 2,60 |
| 67 | $C_6H_5$ | $C_6H_5$ | $CH_3$ | 201 | 2,60 |
| 68 | $COOC_2H_5$ | $C_6H_5$ | $CH_3$ | 135 | 2,83 |
| 69 | $OCH_3$ | H | $CH_3$ | 109 | 2,68 |
| 70 | H | $CH_3$ | $CH_3$ | 109 | 2,73 |
| 71 | $CH_2C_6H_5$ | H | $CH_3$ | 110 | 2,72 |
| 72 | $CH_3CH_2$ | $CH_3$ | $CH_3$ | 101 | 2,80 |
| 73 | $CH_3$ | $CH_3-CH-CH_3$ | $CH_3$ | 99 | 2,80 |
| 74 | $C_6H_5$ | $CH_3$ | $CH_3$ | 119 | 2,64 |
| 75 | H | $CH_2-CH_3$ | $CH_3$ | 80 | 2,74 |
| 76 | $CH_3$ | H | $C_2H_5$ | 100-102 | 3,18 |
| 77 | $CH_3$ | H | $CH_2C_6H_5$ | 136 | 4,38 |

**V - Préparation des composés de formule IX**

A une solution toluénique contenant 1 équivalent de dithiolethione oxime II et quelques gouttes de pyridine, on ajoute en refroidissant extérieurement par un bain d'eau glacée, 1,1 équivalent de chlorure d'acétyle. L'agitation est maintenue pendant 2 heures, on verse alors la solution précédente dans de l'eau et on extrait au toluène. La solution toluénique est ensuite lavée à l'eau, séchée sur sulfate de sodium, filtrée, concentrée et le résidu chromatographié sur silicagel. Les o-acyldithiolethione oximes IX sont élués au toluène.

On a rassemblé dans le tableau V les caractéristiques des composés de formule IX.

TABLEAU V

| Code | $R_1$ | $R_2$ | $R''_3$ | Rendement | F° C |
|---|---|---|---|---|---|
| 81 | $CH_3$ | H | $CH_3$ | 50 % | 127 |
| 82 | $C_6H_5$ | H | $CH_3$ | 55 % | 164 |

**VI - Préparation des composés de formule X**

On dissout 1 g de 5-acétyl-4-méthyl-1,2-dithiole-3-thione dans 150 $cm^3$ d'éthanol absolu. On refroidit extérieurement par un bain d'eau glacée et on ajoute alors un excès de cyanoborohydrure. On laisse revenir à température ambiante et on continue à agiter une dizaine d'heures.

On concentre à sec la solution. On reprend alors après refroidissement par quelques ml d'une solution d'acide chlorhydrique dilué. On traite le précipité et la solution par du toluène. La dithiolethione alcool ne se dissout pas. On la purifie par chromatographie sur silicagel. Elle est déposée sur la colonne après dissolution dans le minimum d'acétate d'éthyle; elle est éluée au mélange acétate d'éthyle/ toluène (30/70). On obtient la 5-(1-hydroxyéthyl)-4-méthyl-1,2-dithiole-3-thione sous forme de cristaux jaune clair F = 79-80° C (benzène).

**VII - Préparation des composés de formule XI**

On porte à reflux pendant 3 heures dans l'éthanol absolu un mélange équimolaire d'aniline et de dithiolethioneoxime II 1. La solution noircit immédiatement. On concentre à sec la solution éthanolique. On

chromatographie le résidu sur silicagel. La 5-phényliminométhyl-4-méthyl-1,2-dithiole-3-thione se présente sous forme de cristaux noirs F = 124-125 ° C (éther de pétrole).

**VIII - Préparation des composés de formule XII**

On utilise le mode opératoire de BOBERG et KNOOP (LIEBIGS ANN. CHEM. 1967, 708, 148) :

On dissout 1 g de 5-formyl-4-méthyl-1,2 dithiolethione et 1 g de chlorure de benzhydroxamoyle (GW Perold, AP Steyn et F.K.V. - Von Reiche, J. Am. Chem. Soc. 1957, 79, 462) dans un minimum de toluène sec. On ajoute goutte à goutte sous agitation de la triéthylamine jusqu'à fin de formation de chlorhydrate de triéthylamine. Durant cette addition le mélange est maintenu à la température ambiante. La solution initialement rouge foncé devient incolore. On poursuit l'agitation pendant 3 h, on filtre alors le chlorhydrate et on concentre le filtrat à sec. On reprend le résidu par 100 cc de xylène et l'on porte à reflux pendant 3 h. On concentre à sec; le résidu est cristallisé dans le méthanol. La 5-formyl-4-méthyl-1,2-dithiole-3-one est un solide incolore, F = 84 ° C, Rdt = 65 % ( C = 0 Kbr 1640 cm-1).

**IX - Préparation des composés de formule XIII**

Dans le minimum d'acétone sèche on dissout des quantités équivalentes de l'oxime de la 5-formyl-4-méthyl-dithiolethione et d'acétylène dicarboxylate de méthyle. Après une vingtaine d'heures de repos le composé d'addition de formule XIII 3-/4,5 diméthoxycarbonyl-1,3-dithiole-2-ylidènes/-2-thioxo-butanal-oxime cristallise; il est filtré et de nouveau cristallisé dans l'acétone F = 139 ° C, cristaux rouge foncé, IR : OH 3210 $cm^{-1}$ bande large ; C=0 1720-1740 $cm^{-1}$. RMN $^1H$ ($CH_3D_6O$) ppm : OH (s : 10,68) ; H(CH) (s : 8,42) $CH_3$ (s : 2,82) ; $CH_3$ (COO $CH_3$) (s : 3,92 et 4).

**X - Préparation des composés de formule Ia**

**Exemple : préparation de l'iodure de 5-for-myl-4-méthyl-3 méthylthio-1,2-dithiolylium**

On dissout de la 5-formyl-4-méthyl-1,2- dithiole-3-thione dans un excès d'iodure de méthyle.
On laisse reposer pendant plusieurs jours ; l'ion dithiolylium cristallise.
On filtre et recristallise dans le benzène.
On obtient ainsi l'iodure de 5-formyl-4-méthylthio-1,2-dithiolylium, F = 140-142 ° C.

**XI - Préparation des composés de formule III**

**Exemple A :**

Préparation de la 5 -éthyl-4-phényl-1,2 -dithiole- 3-thione :

Elle est obtenue à partir de la 1 -phényl-2 -butanone commerciale (Aldrich pureté 98%) en appliquant la méthode de Thuillier et Vialle (Bull. Soc. Chim. Fr, 1962, 2187).

La 1,1-bis méthylthio (2 -phényl-1 -pentène-3 -one) obtenue par condensation du sulfure de carbone avec la cétone précédente en présence de tertioamylate de sodium, suivie d'une méthylation par l'iodure de méthyle (Rdt = 80%), est traitée par le pentasulfure de phosphore en solution xylénique. La dithiolethione est isolée suivant la méthode usuelle (refroidissement, lavage par solution de soude puis à l'eau jusqu'à neutralité, séchage sur sulfate de sodium, concentration et chromatographie). Rendement de la sulfuration = 70%.

Ce sont des cristaux orangés ; F = 64 ° C (benzene)

RMN1H (CDCl3, δ ppm/TMS) : 1,35 (t,3H) ; 2,65 (q, 2H) ; 7,25 à 7,65 (m, 5H).

**Exemple B :**

Préparation de la 5-propyl-1,2 -dithiole-3 - thione

Elle est obtenue par sulfuration du butyryl acétate d'éthyle (produit commercial Aldrich pureté 98%) suivant le procédé de L. Legrand et N Lozac'h (Bull. Soc. Chim. Fr, 195, 79).

On porte au reflux une suspension de 0,061 mole de pentasulfure de phosphore et 0,26 mole de soufre dans 250 $cm^3$ de xylène sec. On ajoute goutte à goutte la solution de 0,13 mole de butyrylacétate d'éthyle dissout dans 30 $cm^3$ de xylène sec. On laisse au reflux 15 à 30 minutes. Après refroidissement, la solution xylénique est lavée par une solution d'hydroxyde de sodium diluée, puis à l'eau jusqu'à neutralité. Après séchage sur sulfate de sodium, la 5 -propyl-1,2-dithiole-3-thione est purifiée par chromatographie sur alumine neutre activée (Rdt = 70%) c'est un liquide rouge foncé.
RMN $^1H$ ($CDCl_3$, $\delta$ ppm/TMS : 1,10 (t, 3H) ; 1,87 (m,2H) 2,83 (t, 2H) ; 7,23 (s, 1H).

On donnera ci-après les caractéristiques de dithiolethiones de formule III.

| | $R_1$ | $R_2$ | Couleur | F°C | Solvant de cristallisation |
|---|---|---|---|---|---|
| la 5-éthyl-4-phényl-1,2-dithiole-3-thione | $C_6H_5$ | $CH_3$ | cristaux orange | 64 | Benzène |
| la 4-méthoxy-5-méthyl-1,2-dithiole-3-thione | $CH_3O$ | H | cristaux jaunes | 51 | Ether de pétrole |
| la 5-isobutyl-4-méthyl-1,2-dithiole-3-thione | $CH_3$ | $CH_3$-CH-$CH_3$ | cristaux jaunes orange | 50 | Ether de pétrole |
| la 4-phényl-5-(2-phényl éthyl)-1,2-dithiole-3-thione | $C_6H_5$ | $C_6H_5CH_2$ | cristaux rouge orangé | 101 | Méthanol |
| la 5-benzyl-4-éthoxycarbonyl-1,2-dithiole-3-thione | COOEt | $C_6H_5$ | cristaux marron clair | 37 | Benzène |
| la 5-α-thiénylméthyl-1,2-dithiole-3-thione | H | 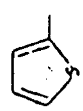 | cristaux jaunes | 98 | Cyclohexane |
| la 4-benzyl-5-éthyl-1,2-dithiole-3-thione | $C_6H_5CH_2$ | $CH_3$ | liquide jaune* | | |
| la 5-propyl-1,2-dithiole-3-thione | H | $CH_3CH_2$ | liquide rouge* | | |

* Liquides non distillés

On a trouvé que les composés de formule I possédaient des propriétés pharmacologiques intéressantes et pouvaient être utilisés en thérapeutique.

- Les composés de formule II et VI ont une activité antimicrobienne importante ;

- les composés de formule II ont une action notable sur les canaux calciques et potassiques et de ce fait présentent entre autres une activité dans le domaine cardio-vasculaire et en gastro-entérologie ainsi qu'une action au niveau du système nerveux central et du système respiratoire.

De plus, certains d'entre eux sont anti-hypertenseurs ou $\beta$-bloquants et sont actifs sur le système respiratoire, plus particulièrement au niveau de la trachée et ont des propriétés antiallergiques dues à leur action broncho-relaxante.

Les produits de l'invention possèdent, tous groupes confondus, des propriétés antiagrégantes plaquettaires.

De plus, les composés de formule I se sont révélés être des capteurs de radicaux libres. Ils peuvent de ce fait trouver des applications comme anti inflammatoires, antiathéromes, antiischémiques, pour lutter contre le vieillissement, comme protecteur hépatique, radioprotecteur et agent anti-cancérigène, en gastro-entérologie, au niveau cardiovasculaire, respiratoire et central, et plus généralement comme antioxydants.

On donnera ci-après des résultats des études toxicologiques effectuées chez la souris.

D'autre part, les composés de formule I exercent un effet sur le glutathion et les enzymes glutathiondépendantes et entraînent les thérapeutiques afférentes (détoxification, pouvoir anticancérigène). De plus, les composés de formule I se sont révélés immunomodulateurs.

**TOXICITE OXIMES DE FORMULE II**

| $R_1$ | $R_2$ | Code | Toxicité en mg/kg | |
|---|---|---|---|---|
| | | | ip (souris) | po (souris) |
| $-(CH_2)_3-$ | | 11 | 200 | 300 |
| H | H | 3 | 50 | 100 |
| H | $C_6H_5$ | 4 | 100 | 300 |
| $C_2H_5$ | $CH_3$ | 9 | > 100 | 300 |
| H | $C_2H_5$ | 17 | 100 | 300 |
| $OCH_3$ | H | 5 | 50 | 50 |

**TOXICITE CETONE DE FORMULE VI**

| $R_1$ | $R_2$ | Code | Toxicité en mg/kg | |
|---|---|---|---|---|
| | | | ip (souris) | po (souris) |
| $CH_3$ | H | 41 | 100 | 100 |
| $CH_3$ | $CH_3$ | 44 | 300 | >300 |
| $C_2H_5$ | $CH_3$ | 50 | 100 | >300 |

Les compositions thérapeutiques selon l'invention peuvent être administrées à l'homme ou aux animaux par voie orale ou parentérale.

Elles peuvent être sous la forme de préparations solides, semi-solides ou liquides. Comme exemple, on peut citer les comprimés, les gélules, les suppositoires, les solutions ou suspensions injectables, ainsi que les formes-retard et les formes implantées à libération lente.

Dans ces compositions, le principe actif est généralement mélangé avec un ou plusieurs excipients pharmaceutiquement acceptables habituels bien connus de l'homme de l'art.

La quantité de principe actif administrée dépend évidemment du patient qui est traité, de la voie d'administration et de la sévérité de la maladie.

**Revendications**

**Revendications pour les Etats contractants suivants : BE, CH, DE, FR, GB, IT, LI, NL**

**1.** Composition pharmaceutique contenant, à titre de principe actif, un composé choisi parmi des composés de formules:

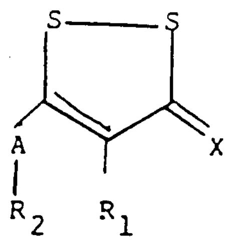 (I) et 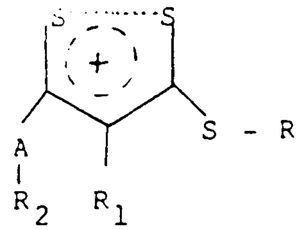 $Y^-$ (Ia)

dans lesquelles :

- X est choisi parmi =S, =O, =N-OH, =N-$R_5$, R5 étant un groupe alkyle en $C_1$-$C_6$ ou aryle, =N-NH-CO-$NH_2$ et =N-NH-CS-$NH_2$, et

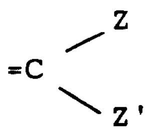 ,

  Z et Z' étant des groupes attracteurs d'électrons,
- A est choisi parmi le groupe >C=N-OH, un groupe de formule >C=N-OR3 (où $R_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$, un groupe aryl(alkyle en $C_1$-$C_6$), un groupe (alkyl en $C_1$-$C_6$) carbonyle et un groupe aryl(alkyl en $C_1$-$C_6$) carbonyle),
  un groupe >C=O, un groupe >C=N-$R_4$, $R_4$ étant un groupe alkyle en $C_1$-$C_6$ ou un groupe aryle, et un groupe CHOH,
- $R_1$ et $R_2$ sont choisis indépendamment l'un de l'autre parmi l'hydrogène, un halogène, un groupe nitro, un groupe nitroso, un groupe thiocyano, un groupe alkyle en $C_1$-$C_6$, un groupe alcényle en $C_2$-$C_6$, un groupe aryle, un groupe aryl(alkyle en $C_1$-$C_6$), un groupe aryl(alcényle en $C_2$-$C_6$), un groupe carboxy, un groupe (alkyl en $C_1$-$C_6$) carbonyle, un groupe arylcarbonyle, un groupe (alcoxy en C1-$C_6$) carbonyle, un groupe (alkoxy en C1-C6) carbonyl(alkyle en $C_1$-$C_6$),un groupe alcoxy en $C_1$-$C_6$, un groupe trifluorométhyle, un groupe amino, un groupe di(alkyl en $C_1$-$C_6$) amino(alkyl en $C_1$-$C_6$), un groupe acylamino de formule -$NHCOC_nH_{2n+1}$ avec n de 0 à 6, un groupe -NH-$CS_nH_{2n+1}$ avec n de 0 à 6, un groupe terpényle, un groupe cyano, un groupe alcynyle en $C_2$-$C_6$, un groupe alcynyle en $C_2$-$C_6$ substitué par un groupe alkyle en $C_1$-$C_6$ ou aryle, un groupe hydroxy(alkyle en $C_1$-$C_6$), un groupe (acyl en $C_1$-$C_6$) oxy(alkyle en $C_1$-$C_6$), un groupe (alkyl en $C_1$-$C_6$)thio, un groupe arylthio,
  ou bien $R_1$ et $R_2$ forment ensemble un groupe alkylène mono ou polycylique en $C_2$-$C_{20}$ comprenant éventuellement un ou plusieurs hétéroatomes, à l'exception du groupe 2,2-diméthyltriméthylène, ou un groupe cycloalkylène en $C_3$-$C_{12}$.
- R est choisi parmi un groupe alkyle en $C_1$-$C_6$,
- $Y^-$ est un anion pharmaceutiquement acceptable.
  et leurs sels pharmaceutiquement acceptables.

**2.** Composition pharmaceutique comprenant, à titre d'ingrédient actif, un composé choisi parmi les composés de formules :

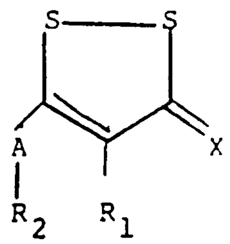

(I)

et

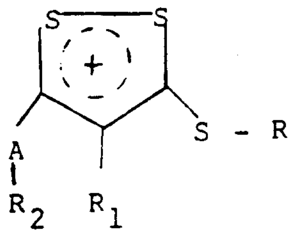

(Ia)

dans lesquelles :

- X est choisi parmi S et O,
- A est choisi parmi le groupe >C=N-OH, un groupe de formule >C=N-OR3 (où $R_3$ est choisi parmi un groupe alkyle en C1-C6, un groupe alkyle en $C_1$-C6 substitué par un ou plusieurs groupes choisis parmi les groupes hydroxy, amino, chloro et alkoxy en $C_1$-C4, un groupe aryl(alkyle en $C_1$-$C_6$), un groupe (alkyl en $C_1$-C6) carbonyle et un groupe aryl(alkyl en $C_1$-C6) carbonyle),
  un groupe C=O, un groupe >C=N-$R_4$, R4 étant un groupe alkyle en $C_1$-C6 ou un groupe aryle, et un groupe CHOH,
- $R_1$ et $R_2$ sont choisis indépendamment l'un de l'autre parmi l'hydrogène, un halogène, un groupe alkyle en $C_1$-$C_6$,un groupe aryle, un groupe aryl(alkyle en $C_1$-$C_6$), un groupe carboxy, un groupe alcoxycarbonyle, et un groupe alcoxy en $C_1$-$C_6$, un groupe trifluorométhyle, un groupe di(alkyl en $C_1$-$C_6$) amino(alkyl en $C_1$-$C_6$) et un groupe acylamino de formule $-NHCOC_nH_{2n+1}$ avec n de 0 à 6, ou bien $R_1$ et R2 forment ensemble un groupe alkylène en $C_2$-$C_{12}$ , à l'exception du groupe 2,2-diméthyltriméthylène, ou un groupe cycloalkylène en $C_3$-$C_{12}$,
- R est choisi parmi un groupe alkyle en $C_1$-$C_6$,
  et leurs sels pharmaceutiquement acceptables.

**3.** Composition selon la revendication 1 comprenant, à titre de principe actif, un composé de formule:

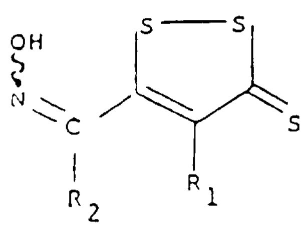

(II)

dans laquelle $R_1$ et $R_2$ ont la signification donnée à la revendication 1.

**4.** Composition selon la revendication 1 comprenant, à titre de principe actif, un composé de formule:

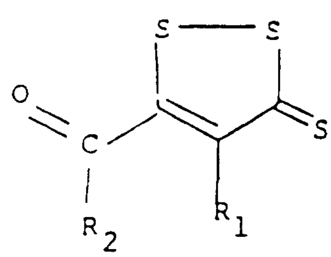

(VI)

dans laquelle $R_1$ et $R_2$ ont la signification donnée à la revendication 1.

**5.** Composition selon la revendication 1 comprenant, à titre de principe actif, un composé de formule:

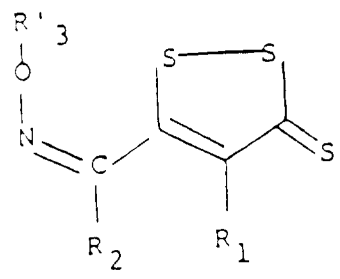

(VII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$).

**6.** Composition selon la revendication 1 comprenant, à titre de principe actif, un composé de formule:

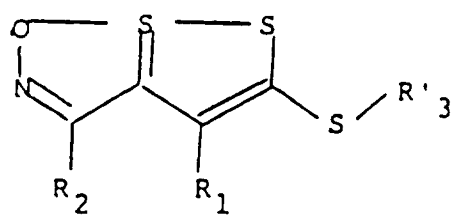

(VIII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$).

**7.** Composition selon la revendication 1 comprenant, à titre de principe actif, un composé de formule:

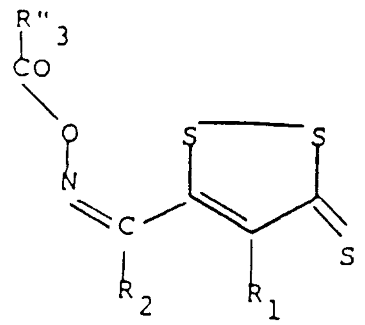

(IX)

dans laquelle $R_1$ et $R_2$ ont la signification donnée à la revendication 1 et $R''_3$ est choisi parmi un atome d'hydrogène, un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-C4 et un groupe aryl(alkyle en $C_1$-$C_6$).

**8.** Composition selon la revendication 1 comprenant, à titre de principe actif, un composé de formule:

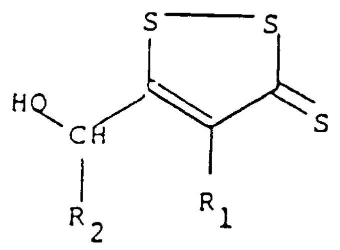

(X)

dans laquelle $R_1$ et $R_2$ ont la signification donnée a la revendication 1.

**9.** Composition selon la revendication 1 comprenant, à titre de principe actif, un composé de formule:

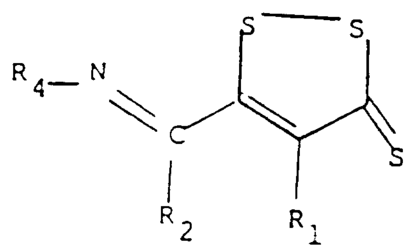

(XI)

dans laquelle $R_1$ et $R_2$ ont la signification donnée à la revendication 1 et $R_4$ représente un groupe alkyle en $C_1$-$C_6$ ou un groupe aryle.

**10.** Composition selon la revendication 1 comprenant, à titre de principe actif, un composé de formule :

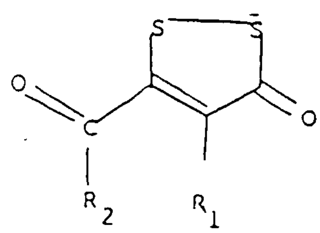

(XII)

dans laquelle $R_1$ et $R_2$ ont la signification donnée à la revendication 1.

**11.** Composés de formule

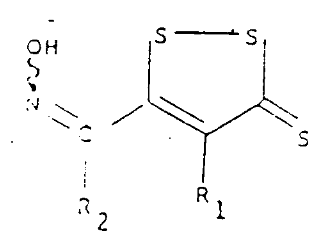

(II)

dans laquelle $R_1$ et $R_2$ ont la signification donnée à la revendication 1.

**12.** Composés de formule

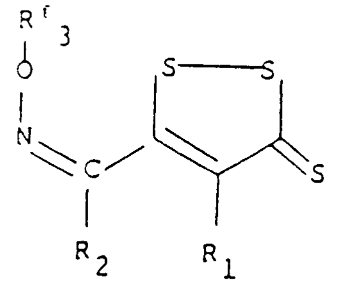

(VII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$).

**13.** Composés de formule

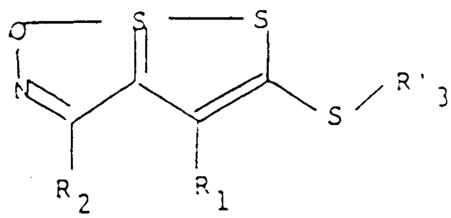

(VIII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$).

**14.** Composés de formule

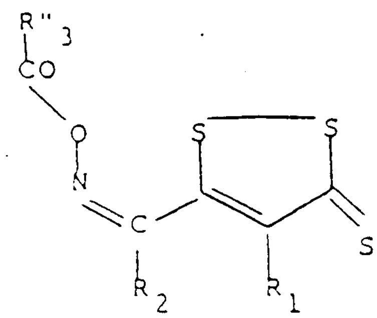

(IX)

dans laquelle $R_1$ et $R_2$ ont la signification donnée à la revendication 1 et $R''_3$ est choisi parmi un atome d'hydrogène, un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$).

15. Composés de formule

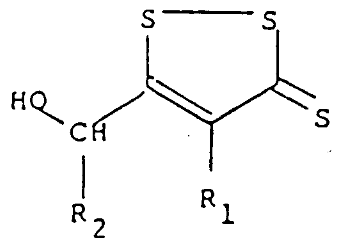

(X)

dans laquelle $R_1$ et $R_2$ ont la signification donnée à la revendication 1.

16. Procédé de préparation de composés selon la revendication 11, caractérisé en ce qu'on fait réagir sur un composé de formule

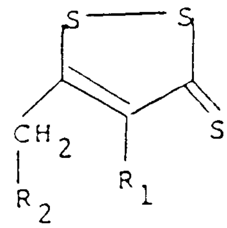

dans laquelle $R_1$ et $R_2$ ont la signification donnée à la revendication 1,
de l'acide nitreux ou du nitrite d'isoamyle.

17. Composés de formule

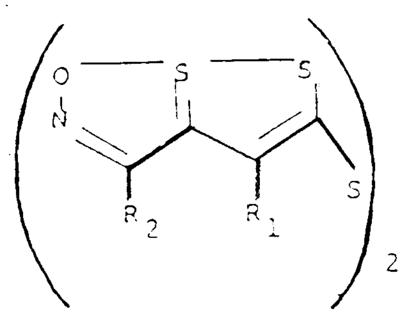

(V)

dans laquelle $R_1$ et $R_2$ ont la signification donnée à la revendication 1.

18. Composés choisis parmi la 5-éthyl-4-phényl-1,2-dithiole-3-thione, la 4-méthoxy-5-méthyl-1,2-dithiole-3-thione, la 5-isobutyl-4-méthyl-1,2-dithiole -3-thione, la 4-phényl-5-(2-phényl éthyl)-1,2-dithiole -3-thione, la 5-benzyl-4-éthoxycarbonyl-1,2-dithiole-3-thione, la 5-$\alpha$-thiénylméthyl-1,2-dithiole-3-thione, la 4-benzyl-5-éthyl-1,2-dithiole-3-thione, et la 5-propyl-1,2-dithiole-3-thione.

**Revendications pour l'Etat contractant suivant : ES**

1. Procédé de préparation d'une composition pharmaceutique contenant, à titre de principe actif, un composé choisi parmi des composés de formules:

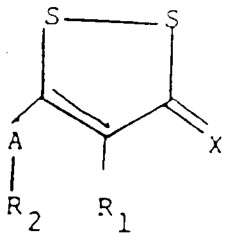 (I) et 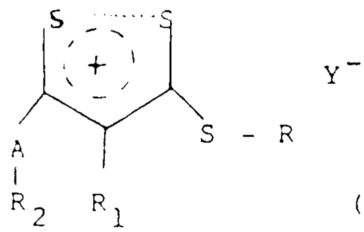 (Ia)

dans lesquelles :

- X est choisi parmi =S, =O, =N-OH, =N-$R_5$, $R_5$ étant un groupe alkyle en $C_1$-$C_6$ ou aryle, =N-NH-CO-$NH_2$ et =N-NH-CS-$NH_2$, et

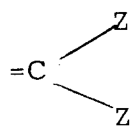 ,

Z et z' étant des groupes attracteurs d'électrons,
- A est choisi parmi le groupe >C=N-OH, un groupe de formule >C=N-OR3 (où $R_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$, un groupe aryl(alkyle en $C_1$-$C_6$), un groupe (alkyl en $C_1$-$C_6$) carbonyle et un groupe aryl(alkyl en $C_1$-$C_6$) carbonyle),
un groupe >C=O, un groupe >C=N-$R_4$, $R_4$ étant un groupe alkyle en $C_1$-$C_6$ ou un groupe aryle, et un groupe CHOH,
- $R_1$ et $R_2$ sont choisis indépendamment l'un de l'autre parmi l'hydrogène, un halogène, un groupe nitro, un groupe nitroso, un groupe thiocyano, un groupe alkyle en $C_1$-$C_6$, un groupe alcényle en $C_2$-$C_6$, un groupe aryle, un groupe aryl(alkyle en $C_1$-$C_6$), un groupe aryl(alcényle en $C_2$-$C_6$), un groupe carboxy, un groupe (alkyl en $C_1$-$C_6$) carbonyle, un groupe arylcarbonyle, un groupe (alcoxy en C1-$C_6$) carbonyle, un groupe (alkoxy en $C_1$-$C_6$) carbonyl(alkyle en $C_1$-$C_6$), un groupe alcoxy en $C_1$-$C_6$, un groupe trifluorométhyle, un groupe amino, un groupe di(alkyl en $C_1$-$C_6$) amino(alkyl en $C_1$-$C_6$), un groupe acylamino de formule -$NHCOC_nH_{2n+1}$ avec n de 0 à 6, un groupe -NH-$CS_nH_{2n+1}$ avec n de 0 à 6, un groupe terpényle, un groupe cyano, un groupe alcynyle en $C_2$-$C_6$, un groupe alcynyle en $C_2$-$C_6$ substitué par un groupe alkyle en $C_1$-$C_6$ ou aryle, un groupe hydroxy(alkyle en $C_1$-$C_6$), un groupe (acyl en $C_1$-$C_6$) oxy(alkyle en $C_1$-$C_6$), un groupe (alkyl en $C_1$-$C_6$)thio, un groupe arylthio,
ou bien $R_1$ et $R_2$ forment ensemble un groupe alkylène mono ou polycylique en $C_2$-$C_{20}$ comprenant éventuellement un ou plusieurs hétéroatomes, à l'exception du groupe 2,2-diméthyltriméthylène, ou un groupe cycloalkylène en $C_3$-$C_{12}$.
- R est choisi parmi un groupe alkyle en $C_1$-$C_6$,
- $Y^-$ est un anion pharmaceutiquement acceptable.
et leurs sels pharmaceutiquement acceptables,
caractérisé par la mise du composé sous une forme pharmaceutiquement acceptable.

2. Procédé de préparation d'une composition pharmaceutique comprenant, à titre d'ingrédient actif, un composé choisi parmi les composés de formules :

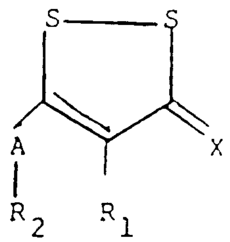

(I)

et

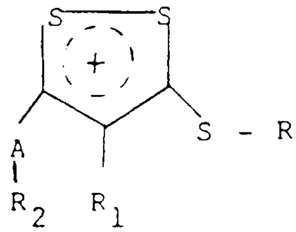

(Ia)

dans lesquelles :

- X est choisi parmi S et O,
- A est choisi parmi le groupe >C = N-OH, un groupe de formule >C = N-OR3 (où $R_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$, un groupe aryl(alkyle en $C_1$-$C_6$), un groupe (alkyl en $C_1$-$C_6$) carbonyle et un groupe aryl(alkyl en $C_1$-$C_6$) carbonyle),
  un groupe >C = O, un groupe >C = N-$R_4$, $R_4$ étant un groupe alkyle en $C_1$-$C_6$ ou un groupe aryle, et un groupe CHOH,
- $R_1$ et R2 sont choisis indépendamment l'un de l'autre parmi l'hydrogène, un halogène, un groupe alkyle en $C_1$-$C_6$,un groupe aryle, un groupe aryl(alkyle en $C_1$-$C_6$), un groupe carboxy, un groupe alcoxycarbonyle, et un groupe alcoxy en $C_1$-$C_6$, un groupe trifluorométhyle, un groupe di(alkyl en $C_1$-$C_6$) amino(alkyl en $C_1$-$C_6$) et un groupe acylamino de formule $-NHCOC_nH_{2n+1}$ avec n de 0 à 6, ou bien $R_1$ et $R_1$ forment ensemble un groupe alkylène en $C_2$-$C_{12}$ , à l'exception du groupe 2,2-diméthyltriméthylène, ou un groupe cycloalkylène en $C_3$-$C_{12}$,
- R est choisi parmi un groupe alkyle en $C_1$-$C_6$,
  et leurs sels pharmaceutiquement acceptables,
  caractérisé par la mise du composé sous une forme pharmaceutiquement acceptable.

**3.** Procédé de préparation selon la revendication 1 d'une composition comprenant, à titre de principe actif, un composé de formule:

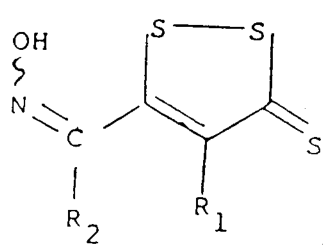

(II)

dans laquelle $R_1$ et $R_2$ ont la signification donnée à la revendication 1,
caractérisé par la mise du composé sous une forme pharmaceutiquement acceptable.

**4.** Procédé de préparation, selon la revendication 1, d'une composition comprenant, à titre de principe actif, un composé de formule:

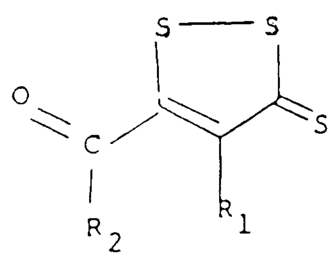

(VI)

dans laquelle $R_1$ et $R_2$ ont la signification donnée à la revendication 1, caractérisé par la mise du composé sous une forme pharmaceutiquement acceptable.

**5.** Procédé de préparation selon la revendication 1 d'une composition comprenant, à titre de principe actif, un composé de formule:

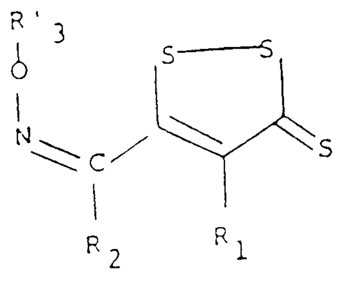

(VII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$), caractérisé par la mise du composé sous une forme pharmaceutiquement acceptable.

**6.** Procédé de préparation, selon la revendication 1, d'une composition comprenant, à titre de principe actif, un composé de formule:

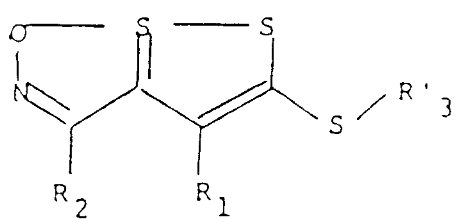

(VIII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$), caractérisé par la mise du composé sous une forme pharmaceutiquement acceptable.

**7.** Procédé de préparation, selon la revendication 1, d'une composition comprenant, à titre de principe actif, un composé de formule:

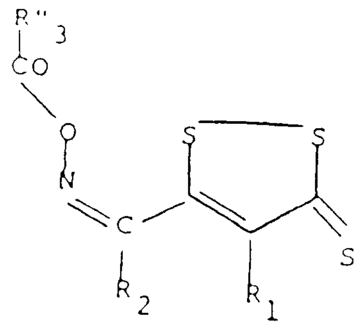

(IX)

dans laquelle $R_1$ et $R_2$ ont la signification donnée à la revendication 1 et $R''_3$ est choisi parmi un atome d'hydrogène, un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-C4 et un groupe aryl(alkyle en $C_1$-$C_6$),
caractérisé par la mise du composé sous une forme pharmaceutiquement acceptable.

**8.** Procédé de préparation, selon la revendication 1, d'une composition comprenant, à titre de principe actif, un composé de formule:

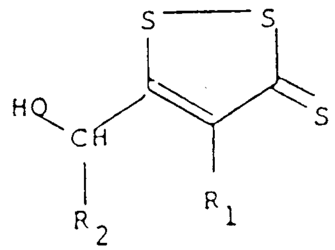

(X)

dans laquelle $R_1$ et $R_2$ ont la signification donnée a la revendication 1,
caractérisé par la mise du composé sous une forme pharmaceutiquement acceptable.

**9.** Procédé de préparation, selon la revendication 1, d'une composition comprenant, à titre de principe actif, un composé de formule:

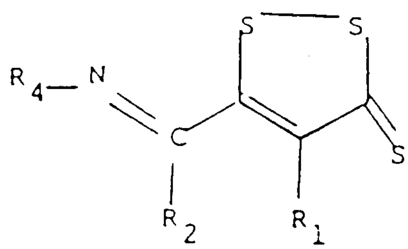

(XI)

dans laquelle $R_1$ et $R_2$ ont la signification donnée à la revendication 1 et $R_4$ représente un groupe alkyle en $C_1$-$C_6$ ou un groupe aryle,
caractérisé par la mise du composé sous une forme pharmaceutiquement acceptable.

**10.** Procédé de préparation, selon la revendication 1, d'une composition comprenant, à titre de principe actif, un composé de formule :

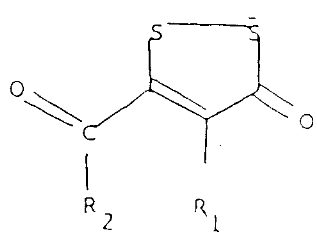

(XII)

dans laquelle $R_1$ et $R_2$ ont la signification donnée à la revendication 1,
caractérisé par la mise du composé sous une forme pharmaceutiquement acceptable.

**11.** Procédé de préparation de composés de formule:

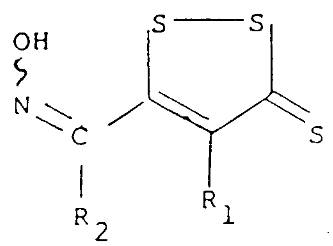

(II)

dans laquelle $R_1$ et $R_2$ ont la signification donnée à la revendication 1,
caractérisé en ce qu'on fait réagir sur un composé de formule

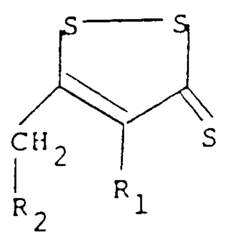

(III)

dans laquelle $R_1$ et $R_2$ ont la signification donnée à la revendication 1,
de l'acide nitreux ou de nitrite d'isoamyle.

**12.** Procédé de préparation de composés de formule

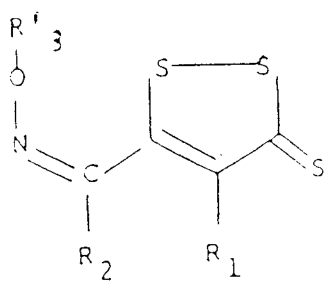

(VII)

dans laquelle $R_1$ et $R_2$ ont la signification donnée à la revendication 1 et $R'_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$),
caractérisé en ce que l'on effectue une alkylation d'une oxime de formule II

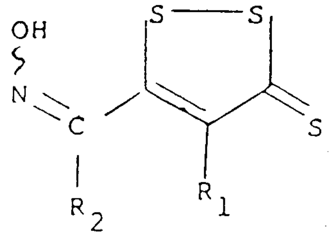

(II)

à l'aide d'un halogénure de formule

R'$_3$-Hal

dans laquelle R'$_3$ a la définition donnée ci-dessus, en présence d'éthylate de sodium.

**13.** Procédé de préparation de composés de formule

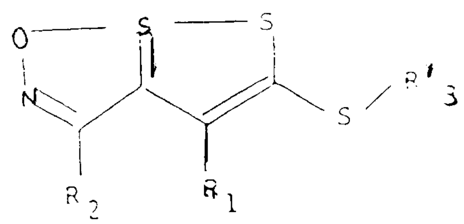

(VIII)

dans laquelle $R_1$ et $R_2$ ont la signification donnée à la revendication 1 et R'$_3$ est choisi parmi un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$),
caractérisé en ce que l'on effectue une alkylation d'une oxime de formule II :

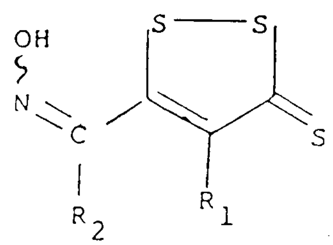

(II)

à l'aide d'un halogénure de formule

R'$_3$-Hal

dans laquelle R'$_3$ a la définition donnée ci-dessus, en présence d'une solution aqueuse d'hydroxyde de sodium.

**14.** Procédé de préparation de composés de formule

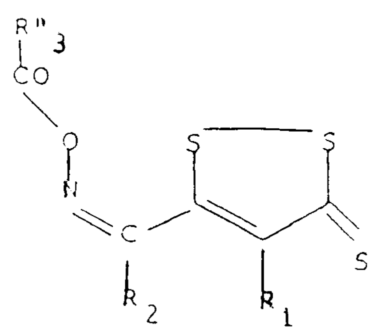

(IX)

dans laquelle $R_1$ et $R_2$ ont la signification donnée à la revendication 1 et R''$_3$ est choisi parmi un atome d'hydrogène, un groupe alkyle en $C_1$-$C_6$, un groupe alkyle en $C_1$-$C_6$ substitué par un ou plusieurs groupes choisis parmi les groupes hydroxy, amino, chloro et alkoxy en $C_1$-$C_4$ et un groupe aryl(alkyle en $C_1$-$C_6$),

caractérisé en ce que l'on effectue une acylation d'une oxime de formule II

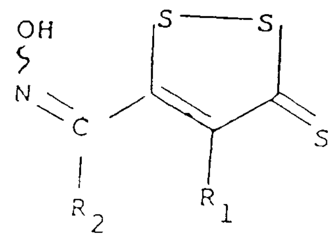

(II)

par un chlorure d'acide de formule

$R''_3COCl$

$R''_3$ ayant la signification donnée ci-dessus,
en milieu toluénique en présence du pyridine.

**15.** Procédé de préparation de composés de formule

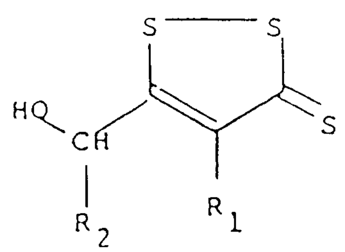

(X)

dans laquelle $R_1$ et $R_2$ ont la signification donnée à la revendication 1,
caractérisé en ce que l'on réduit un composé de formule VI

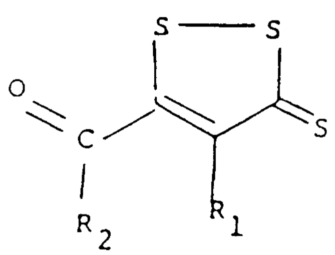

(VI)

**16.** Procédé de préparation de composés de formule :

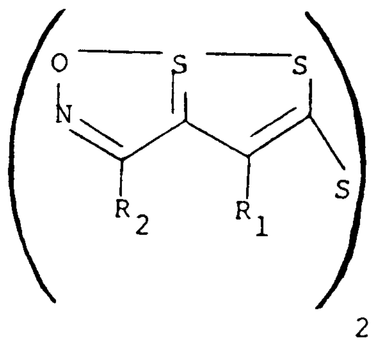

(V)

dans laquelle $R_1$ et $R_2$ ont la signification donnée à la revendication 1, caractérisé en ce que l'on fait réagir sur un composé de formule

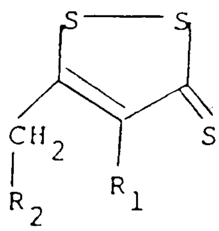

(III)

du nitrite de sodium en milieu acétique glacial.

**Claims**

**Claims for the following Contracting States : BE, CH, DE, FR, GB, IT, LI, NL**

**1.** Pharmaceutical composition containing, as active principle, a compound selected from the compounds of formulae

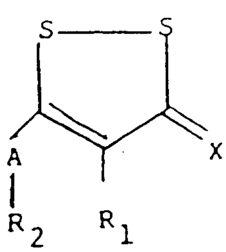

(I)

and

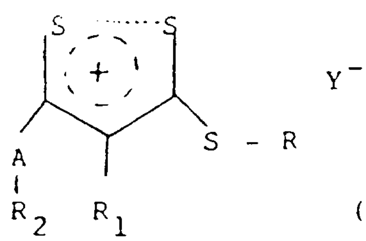

(Ia)

wherein

- X is selected from =S, =O, =N-OH, =N-$R_5$, $R_5$ being a $C_{1-6}$-alkyl or aryl group, =N-NH-CO-$NH_2$ and =N-NH-CS-$NH_2$, and

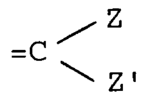

,

Z and Z' being electron attracting groups,

- A is selected from the groups $>C=N\text{-}OH$, a group of formula $>C=N\text{-}OR3$ (where $R_3$ is selected from a $C_{1-6}$-alkyl group, a $C_{1-6}$-alkyl group substituted by one or more groups selected from the hydroxy, amino, chloro and $C_{1-4}$-alkoxy, an aryl ($C_{1-6}$alkyl) group, a ($C_{1-6}$alkyl)-carbonyl and an aryl($C_{1-6}$alkyl)-carbonyl group),
  a group $>C=O$, a group $>C=N\text{-}R_4$, wherein $R_4$ is a $C_{1-6}$-alkyl or an aryl group, and a CHOH group,
- $R_1$ and $R_2$ independently of one another represent hydrogen, a halogen, a nitro group, a nitroso group, a thiocyano group, a $C_{1-6}$-alkyl group, a $C_{2-6}$-alkenyl, an aryl group, an aryl($C_{1-6}$alkyl) group, an aryl($C_{2-6}$alkenyl) group, a carboxy group, a ($C_{1-6}$alkyl)carbonyl group, an arylcarbonyl group, a ($C_{1-6}$alkoxy)carbonyl group, a ($C_{1-6}$alkoxy)carbonyl($C_{1-6}$alkyl) group, a $C_{1-6}$-alkoxy group, a trifluoromethyl group, an amino group, a di($C_{1-6}$alkyl)amino($C_{1-6}$alkyl) group, an acylamino group of the formula $-NHCOC_nH_{2n+1}$ wherein n denotes from 0 to 6, an $-NH\text{-}CS_nH_{2n+1}$ group wherein n denotes from 0 to 6, a terpenyl group, a cyano group, a $C_{2-6}$-alkynyl group, a $C_{2-6}$-alkynyl group substituted by a $C_{1-6}$-alkyl or -aryl group, a hydroxy($C_{1-6}$alkyl) group, a ($C_{1-6}$acyl)oxy($C_{1-6}$alkyl) group, a ($C_{1-6}$alkyl)thio group, an arylthio group,
  or $R_1$ and $R_2$ together form a mono- or polycyclic $C_{2-20}$-alkylene group optionally having one or more heteroatoms, with the exception of the 2,2-dimethyltrimethylene group, or a $C_{3-12}$-cycloalkylene group,
- R is a $C_{1-6}$-alkyl group,
- $Y^-$ is a pharmaceutically acceptable anion,
  and the pharmaceutically acceptable salts thereof.

**2.** Pharmaceutical composition containing, as active ingredient, a compound selected from the compounds of the formulae

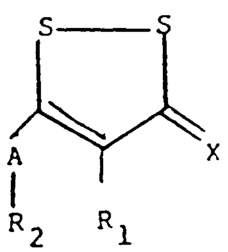

(I) and

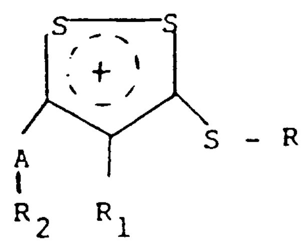

(Ia)

wherein

- X is selected from S and O,
- A may denote the group $>C=N\text{-}OH$, a group of the formula $>C=N\text{-}OR3$
  (wherein $R_3$ is chosen from a $C_{1-6}$-alkyl group, a $C_{1-6}$-alkyl group substituted by one or more groups selected from the hydroxy, amino, chloro and $C_{1-4}$-alkoxy group, an aryl($C_{1-6}$alkyl) group, a ($C_{1-6}$alkyl)carbonyl group and an aryl($C_{1-6}$alkyl)carbonyl group),
  a group $>C=O$, a group $>C=N\text{-}R_4$, wherein $R_4$ is a $C_{1-6}$-alkyl group or an aryl group, and a group CHOH,
- $R_1$ and $R_2$ independently of each other may denote hydrogen, a halogen, a $C_{1-6}$-alkyl group, an aryl group, an aryl($C_{1-6}$alkyl) group, a carboxy group, an alkoxycarbonyl group, and a $C_{1-6}$-alkoxy group, a trifluoromethyl group, a di($C_{1-6}$alkyl)amino($C_{1-6}$alkyl) group and an acylamino group of the formula $-NHCOC_nH_{2n+1}$ wherein n denotes from 0 to 6, or $R_1$ and $R_2$ together form a $C_{2-12}$-alkylene group, with the exception of the 2,2-dimethyltrimethylene group, or a $C_{3-12}$-cycloalkylene group,
- R denotes a $C_{1-6}$-alkyl group,
  and the pharmaceutically acceptable salts thereof.

3. Composition according to claim 1 comprising, as active principle, a compound of the formula

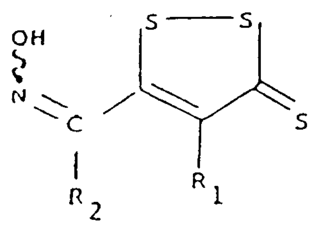

(II)

wherein $R_1$ and $R_2$ have the meanings given in claim 1.

4. Composition according to claim 1 comprising, as active principle, a compound of the formula

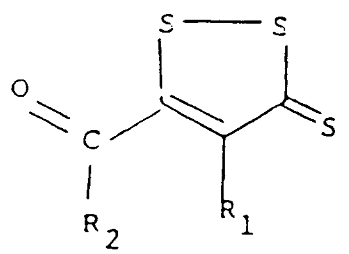

(VI)

wherein $R_1$ and $R_2$ have the meanings given in claim 1.

5. Composition according to claim 1 comprising, as active principle, a compound of formula:

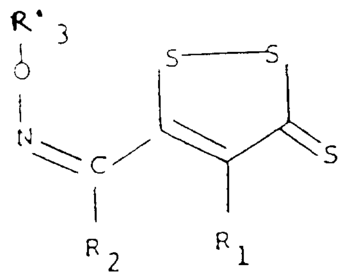

(VII)

wherein $R_1$ and $R_2$ have the meanings given in claim 1 and $R'_3$ may denote a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group.

6. Composition according to claim 1 containing, as active principle, a compound of the formula:

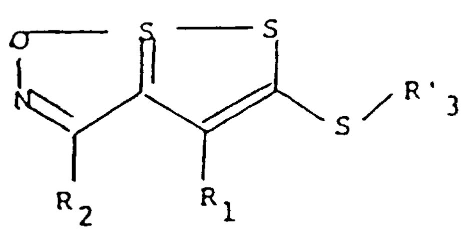

(VIII)

wherein $R_1$ and $R_2$ have the meanings given in claim 1 and $R'_3$ may denote a $C_{1-6}$-alkyl group, a $C_{1-6}$-alkyl group substituted by one or more groups selected from the hydroxy, amino, chloro and

$C_{1-4}$-alkoxy groups, or an aryl($C_{1-6}$alkyl) group.

**7.** Composition according to claim 1 containing, as active principle, a compound of formula

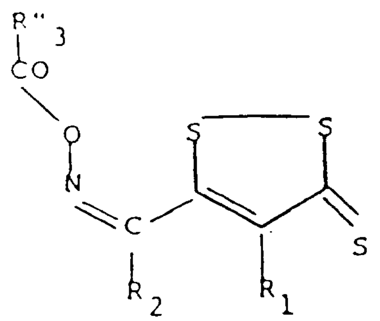

(IX)

wherein $R_1$ and $R_2$ have the meanings given in claim 1 and $R''_3$ may denote a hydrogen atom, a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group.

**8.** Composition according to claim 1 containing, as active principle, a compound of formula

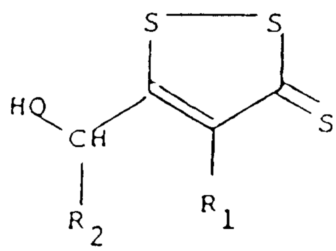

(X)

wherein $R_1$ and $R_2$ have the meanings given in claim 1.

**9.** Composition according to claim 1 containing, as active principle, a compound of formula

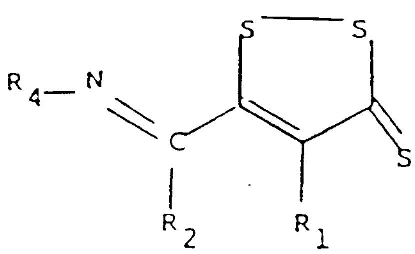

(XI)

wherein $R_1$ and $R_2$ have the meanings given in claim 1 and $R_4$ represents $C_{1-6}$-alkyl group or an aryl group.

**10.** Composition according to claim 1 containing, as active principle, a compound of formula:

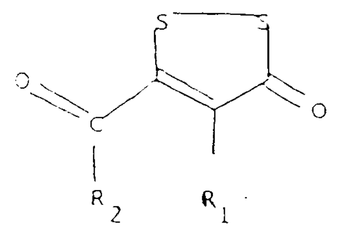

(XII)

wherein $R_1$ and $R_2$ have the meanings given in claim 1.

**11.** Compounds of formula

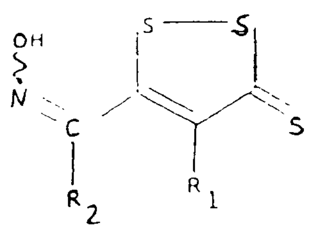

(II)

wherein $R_1$ and $R_2$ have the meanings given in claim 1.

**12.** Compounds of formula

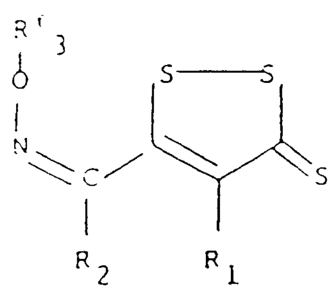

(VII)

wherein $R_1$ and $R_2$ have the meanings given in claim 1 and $R'_3$ may denote a $C_{1-6}$-alkyl group, a $C_{1-6}$-alkyl group substituted by one or more groups selected from among the hydroxy, amino, chloro and $C_{1-4}$-alkoxy groups, or an aryl($C_{1-6}$alkyl) group.

**13.** Compounds of formula

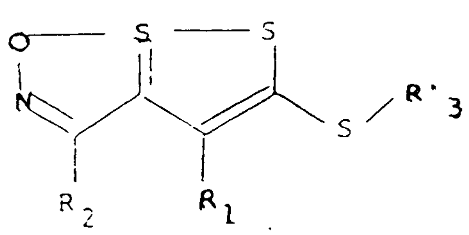

(VIII)

wherein $R_1$ and $R_2$ have the meanings given in claim 1 and $R'_3$ may denote a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group.

**14.** Compounds of formula

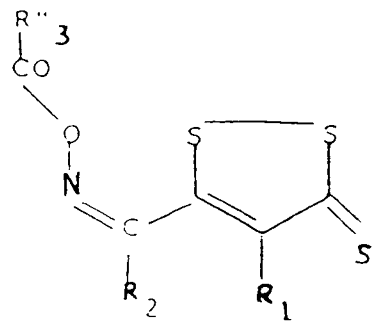

(IX)

wherein $R_1$ and $R_2$ have the meanings given in claim 1 and $R''_3$ may denote a hydrogen atom, a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group.

**15.** Compounds of formula

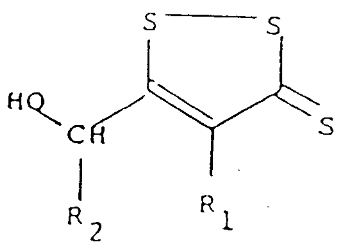

(X)

wherein $R_1$ and $R_2$ have the meanings given in claim 1.

**16.** Process for preparing compounds according to claim 11, characterised in that a compound of formula

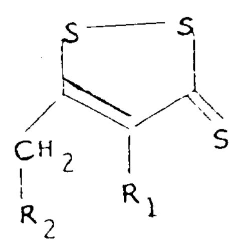

wherein $R_1$ and $R_2$ have the meanings given in claim 1, is reacted with nitrous acid or isoamyl nitrite.

**17.** Compounds of formula

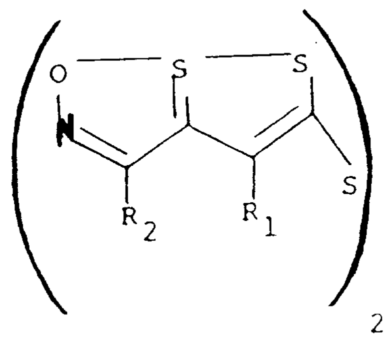

(V)

wherein $R_1$ and $R_2$ have the meanings given in claim 1.

**18.** Compounds selected from 5-ethyl-4-phenyl-1,2-dithiole-3-thione, 4-methoxy-5-methyl-1,2-dithiole-3-thione, 5-isobutyl-4-methyl-1,2-dithiole-3-thione, 4-phenyl-5-(2-phenylethyl)-1,2-dithiole-3-thione, 5-benzyl-4-ethoxycarbonyl-1,2-dithiole-3-thione, 5-$\alpha$-thienylmethyl-1,2-dithiole-3-thione, 4-benzyl-5-ethyl-1,2-dithiole-3-thione, 5-propyl-1,2-dithiole-3-thione.

**Claims for the following Contracting State : ES**

**1.** Process for preparing a pharmaceutical composition containing, as active principle, a compound selected from the compounds of formulae

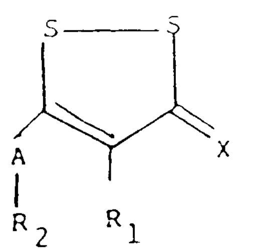

(I)

and

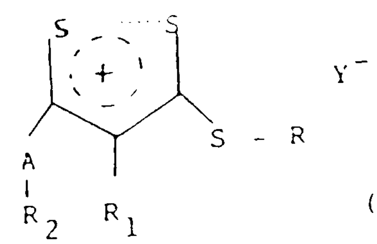

(Ia)

wherein

- X is selected from =S, =O, -N-OH, =N-$R_5$, $R_5$ being a $C_{1-6}$-alkyl or aryl group, =N-NH-CO-$NH_2$ and =N-NH-CS-$NH_2$, and

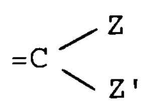

,

Z and Z' being electron attracting groups,
- A is selected from the groups >C=N-OH, a group of formula >C=N-OR3 (where $R_3$ is selected from a $C_{1-6}$-alkyl group, a $C_{1-6}$-alkyl group substituted by one or more groups selected from the hydroxy, amino, chloro and $C_{1-4}$-alkoxy, an aryl ($C_{1-6}$alkyl) group, a ($C_{1-6}$alkyl)-carbonyl and an aryl($C_{1-6}$alkyl)-carbonyl group), a group >C=O, a group >C=N-$R_4$, wherein $R_4$ is a $C_{1-6}$-alkyl or an aryl group, and a CHOH group,
- $R_1$ and $R_2$ independently of one another represent hydrogen, a halogen, a nitro group, a nitroso group, a thiocyano group, a $C_{1-6}$-alkyl group, a $C_{2-6}$-alkenyl, an aryl group, an aryl($C_{1-6}$alkyl) group, an aryl($C_{2-6}$alkenyl) group, a carboxy group, a ($C_{1-6}$alkyl)carbonyl group, an arylcarbonyl group, a ($C_{1-6}$alkoxy)carbonyl group, a ($C_{1-6}$alkoxy)carbonyl($C_{1-6}$alkyl) group, a $C_{1-6}$-alkoxy group, a trifluoromethyl group, an amino group, a di($C_{1-6}$alkyl)amino($C_{1-6}$alkyl) group, an acylamino group of the formula $-NHCOC_nH_{2n+1}$ wherein n denotes from 0 to 6, an $-NH-CS_nH_{2n+1}$

group wherein n denotes from 0 to 6, a terpenyl group, a cyano group, a $C_{2-6}$-alkynyl group, a $C_{2-6}$-alkynyl group substituted by a $C_{1-6}$-alkyl or -aryl group, a hydroxy($C_{1-6}$alkyl) group, a ($C_{1-6}$acyl)oxy($C_{1-6}$alkyl) group, a ($C_{1-6}$alkyl)thio group, an arylthio group, or $R_1$ and $R_2$ together form a mono- or polycyclic $C_{2-20}$-alkylene group optionally having one or more heteroatoms, with the exception of the 2,2-dimethyltrimethylene group, or a $C_{3-12}$-cycloalkylene group,

- R is a $C_{1-6}$-alkyl group,
- $Y^-$ is a pharmaceutically acceptable anion,

and the pharmaceutically acceptable salts thereof, characterised in that the compound is put into a pharmaceutically acceptable form.

**2.** Process for preparing a pharmaceutical composition containing, as active ingredient, a compound selected from the compounds of the formulae

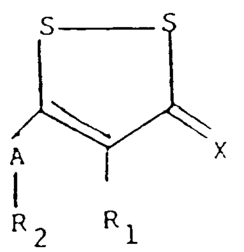

(I)

and

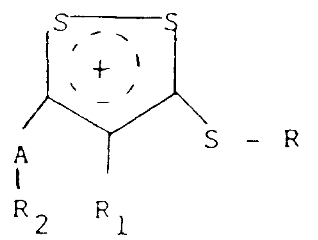

(Ia)

wherein

- X is selected from S and O,
- A may denote the group $>C=N\text{-}OH$, a group of the formula $>C=N\text{-}OR3$ (wherein $R_3$ is chosen from a $C_{1-6}$-alkyl group, a $C_{1-6}$-alkyl group substituted by one or more groups selected from the hydroxy, amino, chloro and $C_{1-4}$-alkoxy group, an aryl($C_{1-6}$alkyl) group, a ($C_{1-6}$alkyl)carbonyl group and an aryl($C_{1-6}$alkyl)carbonyl group), a group $>C=O$, a group $>C=N\text{-}R_4$, wherein $R_4$ is a $C_{1-6}$-alkyl group or an aryl group, and a group CHOH,
- $R_1$ and $R_2$ independently of each other may denote hydrogen, a halogen, a $C_{1-6}$-alkyl group, an aryl group, an aryl($C_{1-6}$alkyl) group, a carboxy group, an alkoxycarbonyl group, and a $C_{1-6}$-alkoxy group, a trifluoromethyl group, a di($C_{1-6}$alkyl)amino($C_{1-6}$alkyl) group and an acylamino group of the formula $\text{-NHCOC}_nH_{2n+1}$ wherein n denotes from 0 to 6, or $R_1$ and $R_2$ together form a $C_{2-12}$-alkylene group, with the exception of the 2,2-dimethyltrimethylene group, or a $C_{3-12}$-cycloalkylene group,
- R denotes a $C_{1-6}$-alkyl group,

and the pharmaceutically acceptable salts thereof, characterised in that the compound is put into a pharmaceutically acceptable form.

**3.** Process according to claim 1 for preparing a composition containing, as active principle, a compound of formula

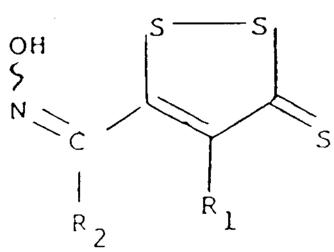

(II)

wherein $R_1$ and $R_2$ have the meanings given in claim 1, characterised in that the compound is put into a pharmaceutically acceptable form.

**4.** Process for preparing, according to claim 1, a composition containing, as active principle, a compound of formula

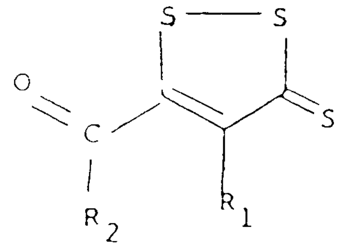

(VI)

wherein $R_1$ and $R_2$ have the meanings given in claim 1, characterised in that the compound is put into a pharmaceutically acceptable form.

**5.** Process for preparing, according to claim 1, a composition containing as active principle a compound of formula

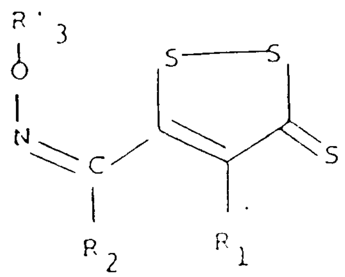

(VII)

wherein $R_1$ and $R_2$ have the meanings given in claim 1 and $R'_3$ may denote a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group,
characterised in that the compound is put into a pharmaceutically acceptable form.

**6.** Process for preparing, according to claim 1, a composition containing as active principle a compound of formula:

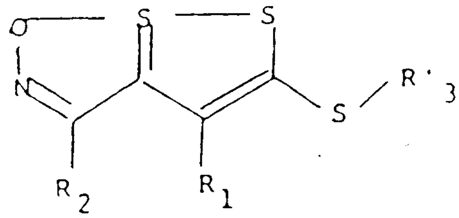

(VIII)

wherein $R_1$ and $R_2$ have the meanings given in claim 1 and $R'_3$ may denote a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group,
characterised in that the compound is put into a pharmaceutically acceptable form.

**7.** Process for preparing, according to claim 1, a composition containing as active principle a compound of formula

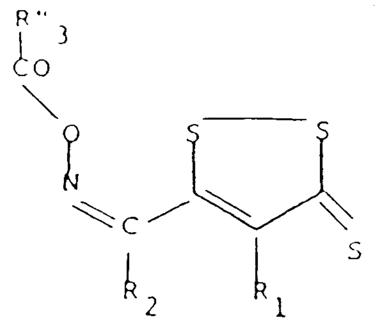

(IX)

wherein $R_1$ and $R_2$ have the meanings given in claim 1 and $R''_3$ may denote a hydrogen atom, a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group,
characterised in that the compound is put into a pharmaceutically acceptable form.

**8.** Process for preparing, according to claim 1, a composition containing as active principle a compound of formula

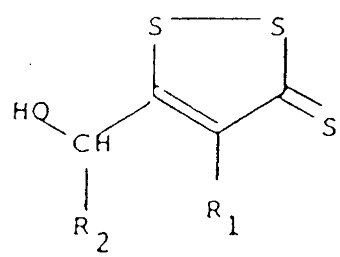

(X)

wherein $R_1$ and $R_2$ have the meanings given in claim 1, characterised in that the compound is put into a pharmaceutically acceptable form.

**9.** Process for preparing, according to claim 1, a composition containing as active principle a compound of formula

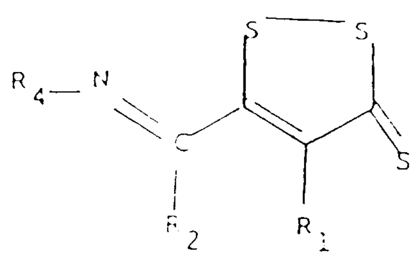

(XI)

wherein $R_1$ and $R_2$ have the meanings given in claim 1 and $R_4$ represents a $C_{1-6}$-alkyl group or an aryl group, characterised in that the compound is put into a pharmaceutically acceptable form.

**10.** Process for preparing, according to claim 1, a composition containing, as active principle, a compound of formula

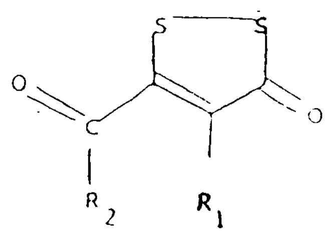

(XII)

wherein $R_1$ and $R_2$ have the meanings given in claim 1, characterised in that the compound is put into a pharmaceutically acceptable form.

**11.** Process for preparing compounds of formula

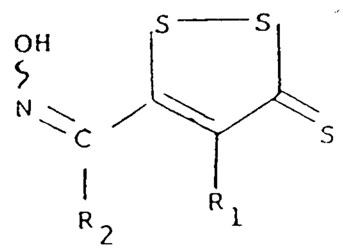

(II)

wherein $R_1$ and $R_2$ have the meanings given in claim 1, characterised in that a compound of formula

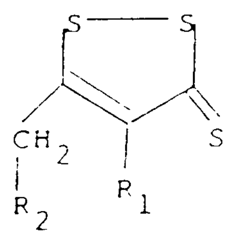

(III)

wherein $R_1$ and $R_2$ have the meanings given in claim 1, is reacted with nitrous acid or isoamyl nitrite.

**12.** Process for preparing compounds of formula

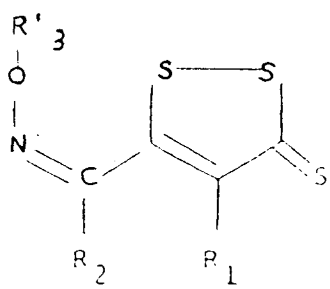

(VII)

wherein $R_1$ and $R_2$ have the meanings given in claim 1 and $R'_3$ may denote a $C_{1-6}$-alkyl group, a $C_{1-6}$-alkyl group substituted by one or more groups selected from among the hydroxy, amino, chloro and $C_{1-4}$-alkoxy groups, or an aryl($C_{1-6}$alkyl) group,
characterised in that an oxime of formula II

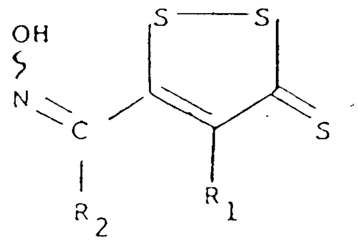

(II)

is alkylated using a halide of formula

R'$_3$-Hal

wherein R'$_3$ is as hereinbefore defined, in the presence of sodium ethoxide.

**13.** Process for preparing compounds of formula

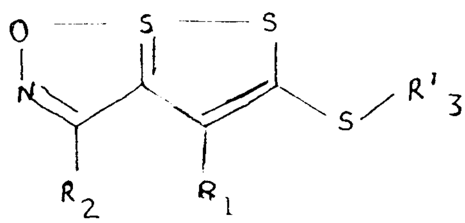

(VIII)

wherein $R_1$ and $R_2$ have the meanings given in claim 1 and R'$_3$ may denote a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group,
characterised in that an oxide of formula II

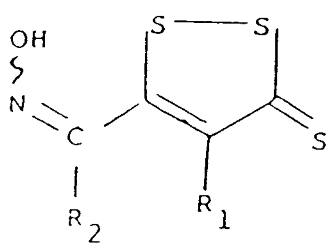

(II)

is alkylated using a halide of formula

R'$_3$-Hal

wherein R'$_3$ is as hereinbefore defined, in the presence of an aqueous sodium hydroxide solution.

**14.** Process for preparing compounds of formula

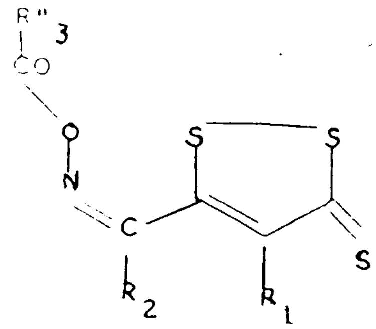

(IX)

wherein $R_1$ and $R_2$ have the meanings given in claim 1 and $R''_3$ may denote a hydrogen atom, a $C_{1-6}$-alkyl group, a $C_{1-6}$-alkyl group substituted by one or more groups selected from the hydroxy, amino, chloro and $C_{1-4}$-alkoxy groups, or an aryl($C_{1-6}$alkyl) group,
characterised in that an oxime of formula II

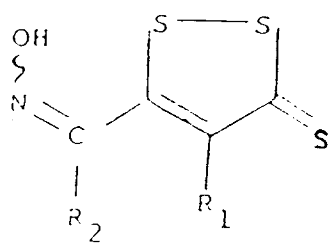

(II)

is acylated by means of an acid chloride of formula

$R''_3COCl$

$R''_3$ being as hereinbefore defined,
in a toluene medium in the presence of pyridine.

**15.** Process for preparing compounds of formula

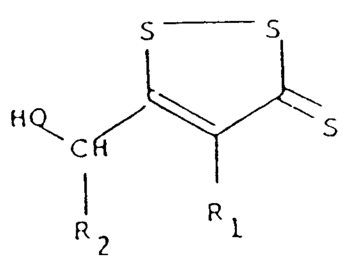

(X)

wherein $R_1$ and $R_2$ have the meanings given in claim 1, characterised in that a compound of formula VI

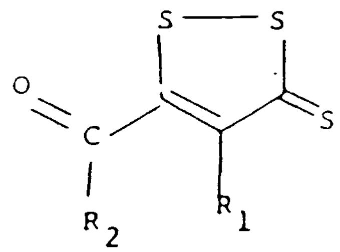

(VI)

is reduced.

**16.** Process for preparing compounds of formula

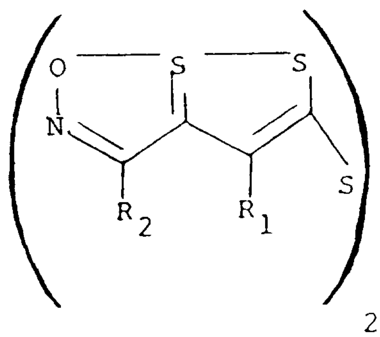

(V)

wherein $R_1$ and $R_2$ have the meanings given in claim 1, characterised in that a compound of formula

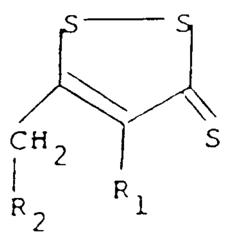

(III)

is reacted with sodium nitrite in a glacial acetic medium.

**Patentansprüche**
**Patentansprüche für folgende Vertragsstaaten : BE, CH, DE, FR, GB, IT, LI, NL**

**1.** Pharmazeutische Zubereitung enthaltend als Wirkstoff eine Verbindung ausgewählt aus Verbindungen der Formeln:

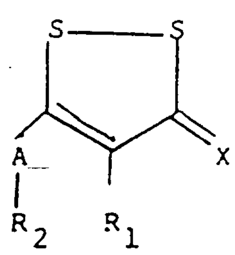

(I)

und

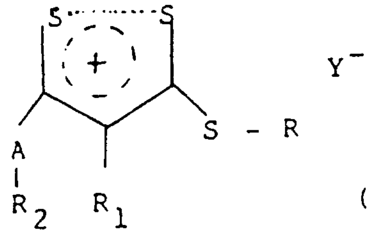

(Ia)

worin:

- X ausgewählt ist aus $=S$, $=O$, $=N\text{-}OH$, $=N\text{-}R_5$, worin $R_5$ eine $C_1$-$C_6$-Alkylgruppe oder eine Arylgruppe darstellt, $=N\text{-}NH\text{-}CO\text{-}NH_2$ und $=N\text{-}NH\text{-}CS\text{-}NH_2$ und

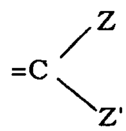,

  worin Z und Z' elektronenanziehende Gruppen darstellen,
- A ausgewählt ist aus der Gruppe $>C=N\text{-}OH$, einer Gruppe der Formel $>C=N\text{-}OR_3$ (worin $R_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, einer Aryl-($C_1$-$C_6$-alkyl)-gruppe, einer ($C_1$-$C_6$-Alkyl)-carbonylgruppe und einer Aryl-($C_1$-$C_6$-alkyl)-carbonylgruppe),
  einer Gruppe $>C=O$, einer Gruppe $>C=N\text{-}R_4$, worin $R_4$ eine $C_1$-$C_6$-Alkylgruppe oder eine Arylgruppe darstellt, und einer Gruppe CHOH,
- $R_1$ und $R_2$ unabhängig voneinander ausgewählt sind aus Wasserstoff, einem Halogenatom, einer Nitrogruppe, einer Nitrosogruppe, einer Thiocyanogruppe, einer $C_1$-$C_6$-Alkylgruppe, einer $C_2$-$C_6$-Alkenylgruppe, einer Arylgruppe, einer Aryl-($C_1$-$C_6$-alkyl)-gruppe, einer Aryl-($C_2$-$C_6$-alkenyl)-gruppe, einer Carboxygruppe, einer ($C_1$-$C_6$-Alkyl)-carbonylgruppe, einer Arylcarbonylgruppe, einer ($C_1$-$C_6$-Alkoxy)-carbonylgruppe, einer ($C_1$-$C_6$-Alkoxy)-carbonyl-($C_1$-$C_6$-alkyl)-gruppe, einer $C_1$-$C_6$-Alkoxygruppe, einer Trifluormethylgruppe, einer Aminogruppe, einer Di($C_1$-$C_6$-alkyl)-amino-($C_1$-$C_6$-alkyl)-gruppe, einer Acylaminogruppe der Formel $-NHCOC_nH_{2n+1}$, worin n einen Wert von 0 bis 6 besitzt, einer Gruppe $-NH\text{-}CS_nH_{2n+1}$, worin n einen Wert von 0 bis 6 aufweist, einer Terpenylgruppe, einer Cyanogruppe, einer $C_2$-$C_6$-Alkinylgruppe, einer $C_2$-$C_6$-Alkinylgruppe, die durch eine $C_1$-$C_6$-Alkylgruppe oder eine Arylgruppe substituiert ist, einer Hydroxy($C_1$-$C_6$-alkyl)-gruppe, einer ($C_1$-$C_6$-Acyl)-oxy-($C_1$-$C_6$-alkyl)-gruppe, einer ($C_1$-$C_6$-Alkyl)-thiogruppe und einer Arylthiogruppe,
  oder $R_1$ und $R_2$ gemeinsam eine monocyclische oder polycyclische $C_2$-$C_{20}$-Alkylengruppe, die gegebenenfalls eines oder mehrere Heteroatome enthält, mit Ausnahme der 2,2-Dimethyltrimethylengruppe, oder eine $C_3$-$C_{12}$-Cycloalkylengruppe bilden,
- R ausgewählt ist aus einer $C_1$-$C_6$-Alkylgruppe,
- $Y^-$ ein pharmazeutisch annehmbares Anion darstellt,
  und deren pharmazeutisch annehmbare Salze.

**2.** Pharmazeutische Zubereitung enthaltend als Wirkstoff eine Verbindung ausgewählt aus den Verbindungen der Formeln:

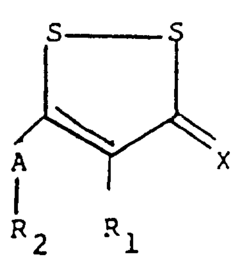

(I)

und

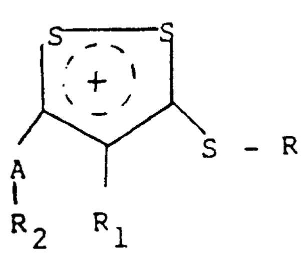

(Ia)

worin:

- X ausgewählt ist aus S und O,
- A ausgewählt ist aus der Gruppe $>C=N\text{-}OH$, einer Gruppe der Formel $>C=N\text{-}OR_3$, (worin $R_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, einer Aryl($C_1$-$C_6$-alkyl)-gruppe, einer ($C_1$-$C_6$-Alkyl)-carbonylgruppe und einer Aryl($C_1$-$C_6$-alkyl)-carbonylgruppe),

einer Gruppe $>C=O$, einer Gruppe $>C=N\text{-}R_4$, worin $R_4$ eine $C_1$-$C_6$-Alkylgruppe oder eine Arylgruppe darstellt, und einer Gruppe CHOH,

- $R_1$ und $R_2$ unabhängig voneinander ausgewählt sind aus Wasserstoff, einem Halogenatom, einer $C_1$-$C_6$-Alkylgruppe, einer Arylgruppe, einer Aryl($C_1$-$C_6$-alkyl)-gruppe, einer Carboxygruppe, einer Alkoxycarbonylgruppe und einer $C_1$-$C_6$-Alkoxygruppe, einer Trifluormethylgruppe, einer Di($C_1$-$C_6$-alkyl)-amino-($C_1$-$C_6$-alkyl)-gruppe und einer Acylaminogruppe der Formel $-NHCOC_nH_{2n+1}$,worin n einen Wert von 0 bis 6 besitzt, oder
  $R_1$ und $R_2$ gemeinsam eine $C_2$-$C_{12}$-Alkylengruppe mit Ausnahme einer 2,2-Dimethyltrimethylengruppe, oder eine $C_3$-$C_{12}$-Cycloalkylengruppe bilden,
- R ausgewählt ist aus einer $C_1$-$C_6$-Alkylgruppe,
  und deren pharmazeutisch annehmbare Salze.

**3.** Zubereitung nach Anspruch 1 enthaltend als Wirkstoff eine Verbindung der Formel:

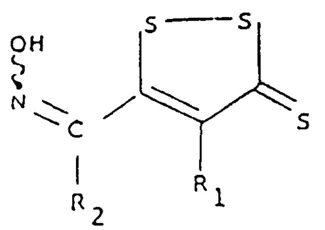

(II)

worin $R_1$ und $R_2$ die in Anspruch 1 angegebenen Bedeutungen besitzen.

**4.** Zubereitung nach Anspruch 1 enthaltend als Wirkstoff eine Verbindung der Formel:

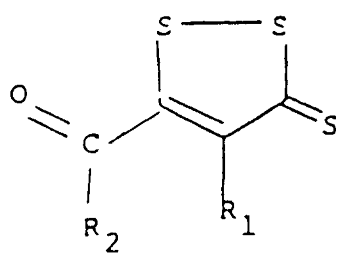

(VI)

worin $R_1$ und $R_2$ die in Anspruch 1 angegebenen Bedeutungen besitzen.

**5.** Zubereitung nach Anspruch 1 enthaltend als Wirkstoff eine Verbindung der Formel:

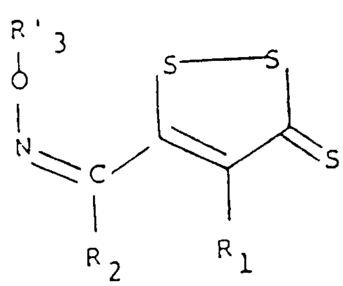

(VII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl($C_1$-$C_6$-alkyl)-gruppe.

**6.** Zubereitung nach Anspruch 1 enthaltend als Wirkstoff eine Verbindung der Formel:

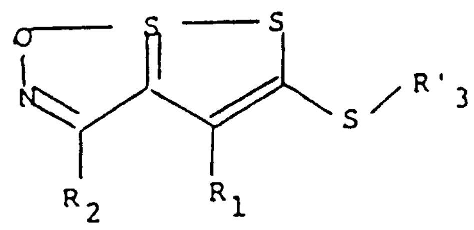

(VIII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl($C_1$-$C_6$-alkyl)-gruppe.

**7.** Zubereitung nach Anspruch 1 enthaltend als Wirkstoff eine Verbindung der Formel:

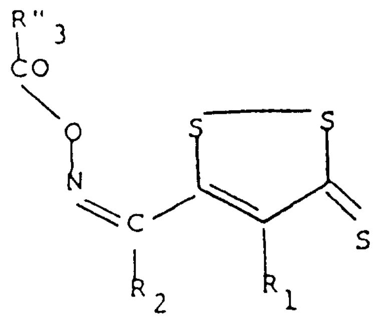

(IX)

in der $R_1$ und $R_2$ die in Anspruch 1 angegebenen Bedeutungen besitzen und $R''_3$ ausgewählt ist aus einem Wasserstoffatom, einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe.

**8.** Zubereitung nach Anspruch 1 enthaltend als Wirkstoff eine Verbindung der Formel:

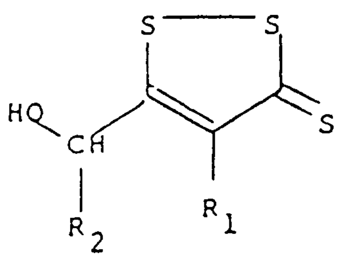

(X)

in der $R_1$ und $R_2$ die in Anspruch 1 angegebenen Bedeutungen besitzen.

**9.** Zubereitung nach Anspruch 1 enthaltend als Wirkstoff eine Verbindung der Formel:

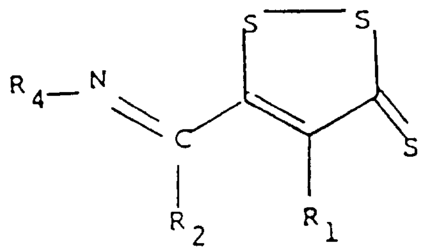

(XI)

in der $R_1$ und $R_2$ die in Anspruch 1 angegebenen Bedeutungen besitzen und $R_4$ eine $C_1$-$C_6$-Alkylgruppe oder eine Arylgruppe darstellt.

**10.** Zubereitung nach Anspruch 1 enthaltend als Wirkstoff eine Verbindung der Formel:

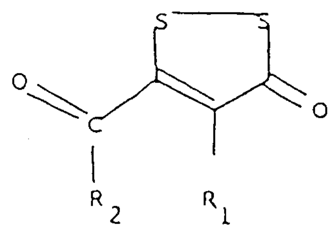

(XII)

in der $R_1$ und $R_2$ die in Anspruch 1 angegebenen Bedeutungen besitzen.

**11.** Verbindungen der Formel

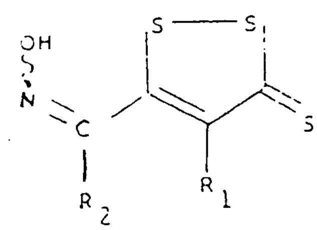

(II)

in der $R_1$ und $R_2$ die in Anspruch 1 angegebenen Bedeutungen besitzen.

**12.** Verbindungen der Formel

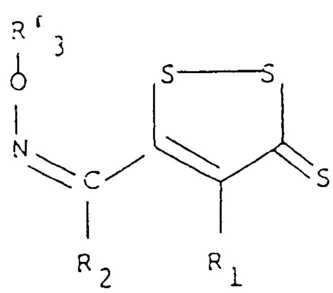

(VII)

in der $R_1$ und $R_2$ die in Anspruch 1 angegebenen Bedeutungen besitzen und $R'_3$ ausgewählt ist aus

einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl($C_1$-$C_6$-alkyl)-gruppe.

**13.** Verbindungen der Formel

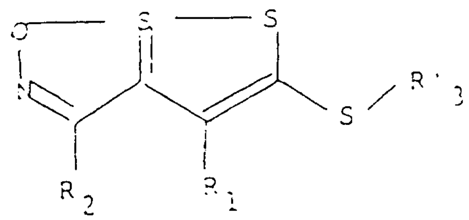

(VIII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe.

**14.** Verbindungen der Formel

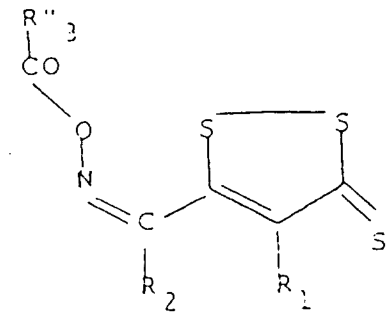

(IX)

in der $R_1$ und $R_2$ die in Anspruch 1 angegebenen Bedeutungen besitzen und $R''_3$ ausgewählt ist aus einem Wasserstoffatom, einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe.

**15.** Verbindungen der Formel

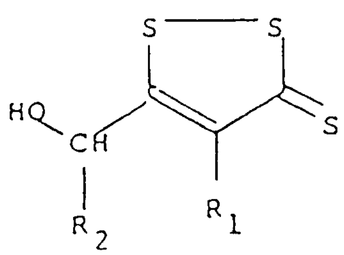

(X)

in der $R_1$ und $R_2$ die in Anspruch 1 angegebenen Bedeutungen besitzen.

**16.** Verfahren zur Herstellung der Verbindungen nach Anspruch 11, **dadurch gekennzeichnet,** daß man eine Verbindung der Formel

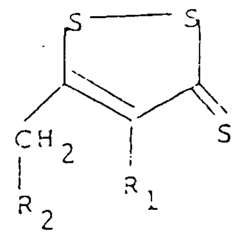

in der $R_1$ und $R_2$ die in Anspruch 1 angegebenen Bedeutungen besitzen, mit salpetriger Säure oder Isoamylnitrit umsetzt.

**17.** Verbindungen der Formel

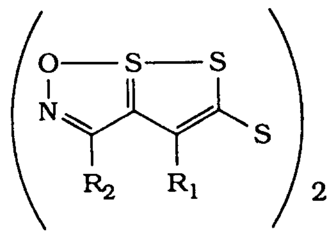

(V)

in der $R_1$ und $R_2$ die in Anspruch 1 angegebenen Bedeutungen besitzen.

**18.** Verbindungen ausgewählt aus 5-Ethyl-4-phenyl-1,2-dithiol-3-thion, 4-Methoxy-5-methyl-1,2-dithiol-3-thion, 5-Isobutyl-4-methyl-1,2-dithiol-3-thion, 4-Phenyl-5-(2-phenyl-ethyl)-1,2-dithiol-3-thion, 5-Benzyl-4-ethoxycarbonyl-1,2-dithiol-3-thion, 5-α-Thienylmethyl-1,2-dithlol-3-thlon, 4-Benzyl-5-ethyl-1,2-dithiol-3-thion und 5-Propyl-1,2-dithiol-3-thion.

**Patentansprüche für folgenden Vertragsstaat : ES**

**1.** Verfahren zur Herstellung einer pharmazeutischen Zubereitung enthaltend als Wirkstoff eine Verbindung ausgewählt aus den Verbindungen der Formeln:

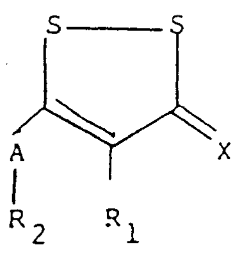

(I)

und

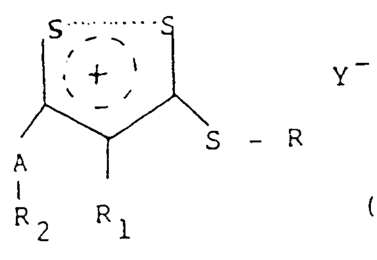

(Ia)

worin:

- X ausgewählt ist aus =S, =O, =N-OH, =N-$R_5$, worin $R_5$ eine $C_1$-$C_6$-Alkylgruppe oder eine Arylgruppe darstellt, =N-NH-CO-$NH_2$ und =N-NH-CS-$NH_2$ und

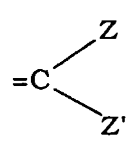

,

worin Z und Z' elektronenanziehende Gruppen darstellen,

- A ausgewählt ist aus der Gruppe $>C=N\text{-}OH$, einer Gruppe der Formel $>C=N\text{-}OR_3$ (worin $R_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen. Aminogruppen, Chloratomen und $C_1$-$C_4$-Alkoxygruppen substituiert ist, einer Aryl-($C_1$-$C_6$-alkyl)-gruppe, einer ($C_1$-$C_6$-Alkyl)-carbonylgruppe und einer Aryl-($C_1$-$C_6$-alkyl)-carbonylgruppe), einer Gruppe $>C=O$, einer Gruppe $>C=N\text{-}R_4$, worin $R_4$ eine $C_1$-$C_6$-Alkylgruppe oder eine Arylgruppe darstellt, und einer Gruppe CHOH,
- $R_1$ und $R_2$ unabhängig voneinander ausgewählt sind aus Wasserstoff, einem Halogenatom, einer Nitrogruppe, einer Nitrosogruppe, einer Thiocyanogruppe, einer $C_1$-$C_6$-Alkylgruppe, einer $C_2$-$C_6$-Alkenylgruppe, einer Arylgruppe, einer Aryl-($C_1$-$C_6$-alkyl)-gruppe, einer Aryl-($C_2$-$C_6$-alkenyl)-gruppe, einer Carboxygruppe, einer ($C_1$-$C_6$-Alkyl)-carbonylgruppe, einer Arylcarbonylgruppe, einer ($C_1$-$C_6$-Alkoxy)-carbonylgruppe, einer ($C_1$-$C_6$-Alkoxy)-carbonyl-($C_1$-$C_6$-alkyl)-gruppe, einer $C_1$-$C_6$-Alkoxygruppe, einer Trifluormethylgruppe, einer Aminogruppe, einer Di($C_1$-$C_6$-alkyl)-amino-($C_1$-$C_6$-alkyl)-gruppe, einer Acylaminogruppe der Formel $-NHCOC_nH_{2n+1}$, worin n einen Wert von 0 bis 6 besitzt, einer Gruppe $-NH\text{-}CS_nH_{2n+1}$, worin n einen Wert von 0 bis 6 aufweist, einer Terpenylgruppe, einer Cyanogruppe, einer $C_2$-$C_6$-Alkinylgruppe, einer $C_2$-$C_6$-Alkinylgruppe, die durch eine $C_1$-$C_6$-Alkylgruppe oder eine Arylgruppe substituiert ist, einer Hydroxy($C_1$-$C_6$-alkyl)-gruppe, einer ($C_1$-$C_6$-Acyl)-oxy-($C_1$-$C_6$-alkyl)-gruppe, einer ($C_1$-$C_6$-Alkyl)-thiogruppe und einer Arylthiogruppe,
  oder $R_1$ und $R_2$ gemeinsam eine monocyclische oder polycyclische $C_2$-$C_{20}$-Alkylengruppe bilden, die gegebenenfalls eines oder mehrere Heteroatome enthält, mit Ausnahme der 2,2-Dimethyltrimethylengruppe, oder eine $C_3$-$C_{12}$-Cycloalkylengruppe bilden,
- R ausgewählt ist aus einer $C_1$-$C_6$-Alkylgruppe,
- $Y^-$ ein pharmazeutisch annehmbares Anion darstellt,
  und von deren pharmazeutisch annehmbaren Salzen,
  **dadurch gekennzeichnet**, daß man die Verbindung in eine pharmazeutisch annehmbare Form bringt.

**2.** Verfahren zur Herstellung einer pharmazeutischen Zubereitung enthaltend als Wirkstoff eine Verbindung ausgewählt aus den Verbindungen der Formeln:

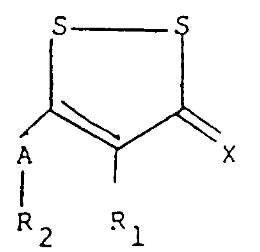
(I)

und

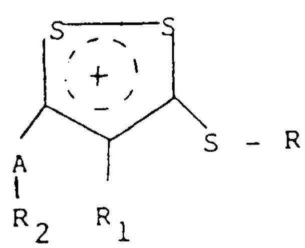
(Ia)

worin:

- X ausgewählt ist aus S und O,
- A ausgewählt ist aus der Gruppe $>C=N\text{-}OH$, einer Gruppe der Formel $>C=N\text{-}OR_3$, (worin $R_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, einer Aryl-($C_1$-$C_6$-alkyl)-gruppe, einer ($C_1$-$C_6$-Alkyl)-carbonylgruppe und einer Aryl-($C_1$-$C_6$-alkyl)-carbonylgruppe), einer Gruppe $>C=O$, einer Gruppe $>C=N\text{-}R_4$, worin $R_4$ eine $C_1$-$C_6$-Alkylgruppe oder eine Arylgruppe darstellt, und einer Gruppe CHOH,
- $R_1$ und $R_2$ unabhängig voneinander ausgewählt sind aus Wasserstoff, einem Halogenatom, einer $C_1$-$C_6$-Alkylgruppe, einer Arylgruppe, einer Aryl($C_1$-$C_6$-alkyl)-gruppe, einer Carboxygruppe, einer Alkoxycarbonylgruppe und einer $C_1$-$C_6$-Alkoxygruppe, einer Trifluormethylgruppe, einer Di($C_1$-$C_6$-alkyl)-amino-($C_1$-$C_6$-alkyl)-gruppe und einer Acylaminogruppe der Formel $-NHCOC_nH_{2n+1}$,worin n einen Wert von 0 bis 6 besitzt, oder
  $R_1$ und $R_2$ gemeinsam eine $C_2$-$C_{12}$-Alkylengruppe mit Ausnahme einer 2,2-Dimethyltrimethylen-

gruppe, oder eine $C_3$-$C_{12}$-Cycloalkylengruppe bilden,

- R ausgewählt ist aus einer $C_1$-$C_6$-Alkylgruppe,

und von deren pharmazeutisch annehmbaren Salzen,

**dadurch gekennzeichnet**, daß man die Verbindung in eine pharmazeutisch annehmbare Form bringt.

**3.** Verfahren nach Anspruch 1 zur Herstellung einer Zubereitung enthaltend als Wirkstoff eine Verbindung der Formel:

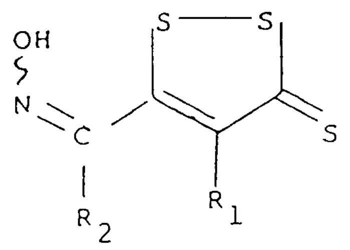

(II)

worin $R_1$ und $R_2$ die in Anspruch 1 angegebenen Bedeutungen besitzen, **dadurch gekennzeichnet**, daß man die Verbindung in eine pharmazeutisch annehmbare Form bringt.

**4.** Verfahren nach Anspruch 1 zur Herstellung einer Zubereitung, die als Wirkstoff eine Verbindung der Formel:

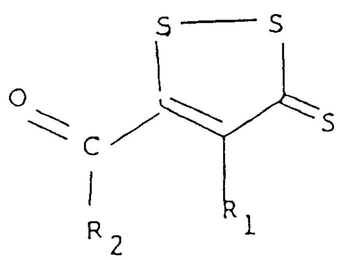

(VI)

enthält, worin $R_1$ und $R_2$ die in Anspruch 1 angegebenen Bedeutungen besitzen, **dadurch gekennzeichnet**, daß man die Verbindung in eine pharmazeutisch annehmbare Form bringt.

**5.** Verfahren nach Anspruch 1 zur Herstellung einer Zubereitung enthaltend als Wirkstoff eine Verbindung der Formel:

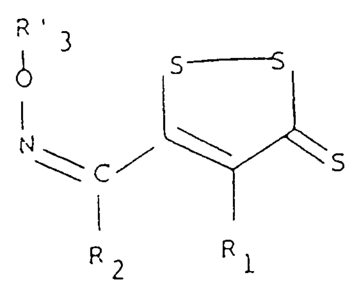

(VII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl($C_1$-$C_6$-alkyl)-gruppe,

**dadurch gekennzeichnet**, daß man die Verbindung in eine pharmazeutisch annehmbare Form bringt.

6. Verfahren nach Anspruch 1 zur Herstellung einer Zubereitung enthaltend als Wirkstoff eine Verbindung der Formel:

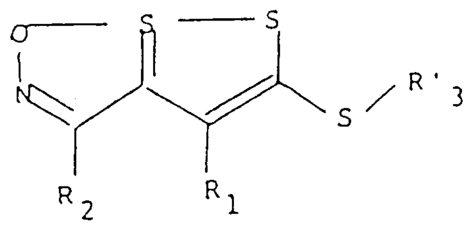

(VIII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe,
**dadurch gekennzeichnet**, daß man die Verbindung in eine pharmazeutisch annehmbare Form bringt.

7. Verfahren nach Anspruch 1 zur Herstellung einer Zubereitung enthaltend als Wirkstoff eine Verbindung der Formel:

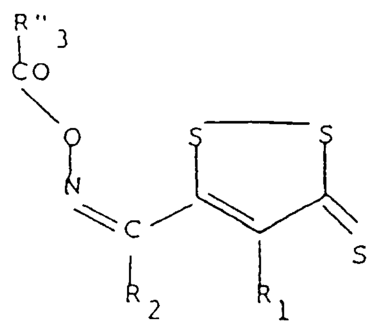

(IX)

in der $R_1$ und $R_2$ die in Anspruch 1 angegebenen Bedeutungen besitzen und $R''_3$ ausgewählt ist aus einem Wasserstoffatom, einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe,
**dadurch gekennzeichnet**, daß man die Verbindung in eine pharmazeutisch annehmbare Form bringt.

8. Verfahren nach Anspruch 1 zur Herstellung einer Zubereitung enthaltend als Wirkstoff eine Verbindung der Formel:

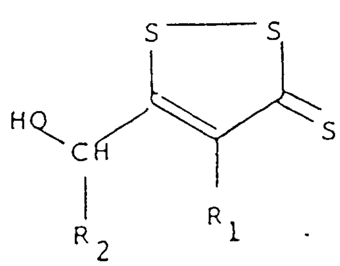

(X)

in der $R_1$ und $R_2$ die in Anspruch 1 angegebenen Bedeutungen besitzen,
**dadurch gekennzeichnet**, daß man die Verbindung in eine pharmazeutisch annehmbare Form bringt.

9. Verfahren nach Anspruch 1 zur Herstellung einer Zubereitung enthaltend als Wirkstoff eine Verbindung der Formel:

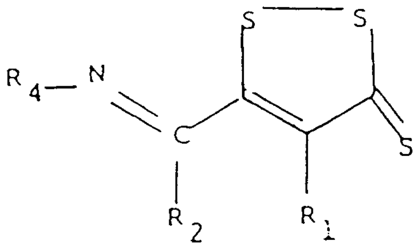

(XI)

in der $R_1$ und $R_2$ die in Anspruch 1 angegebenen Bedeutungen besitzen und $R_4$ eine $C_1$-$C_6$-Alkylgruppe oder eine Arylgruppe darstellt,
**dadurch gekennzeichnet**, daß man die Verbindung in eine pharmazeutisch annehmbare Form bringt.

**10.** Verfahren nach Anspruch 1 zur Herstellung einer Zubereitung enthaltend als Wirkstoff eine Verbindung der Formel:

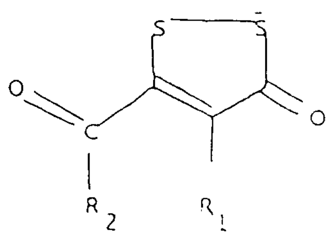

(XII)

in der $R_1$ und $R_2$ die in Anspruch 1 angegebenen Bedeutungen besitzen,
**dadurch gekennzeichnet**, daß man die Verbindung in eine pharmazeutisch annehmbare Form bringt.

**11.** Verfahren zur Herstellung der Verbindungen der Formel:

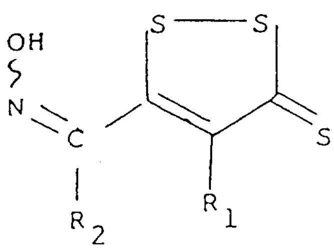

(II)

in der $R_1$ und $R_2$ die in Anspruch 1 angegebenen Bedeutungen besitzen,
**dadurch gekennzeichnet**, daß man eine Verbindung der Formel

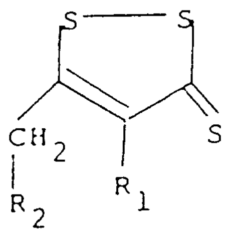

(III)

in der $R_1$ und $R_2$ die in Anspruch 1 angegebenen Bedeutungen besitzen, mit salpetriger Säure oder Isoamylnitrit umsetzt.

**12.** Verfahren zur Herstellung der Verbindungen der Formel

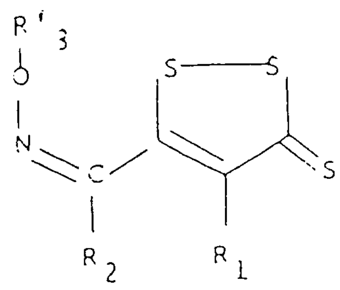

(VII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe,
**dadurch gekennzeichnet**, daß man ein Oxim der Formel II

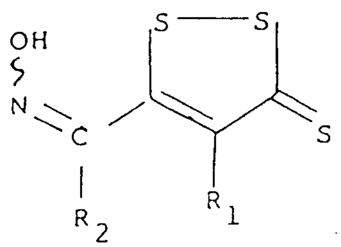

(II)

mit einem Halogenid der Formel

$R'_3$-Hal

worin $R'_3$ die oben angegebenen Bedeutungen besitzt, in Gegenwart von Natriumethylat alkyliert.

**13.** Verfahren zur Herstellung der Verbindungen der Formel

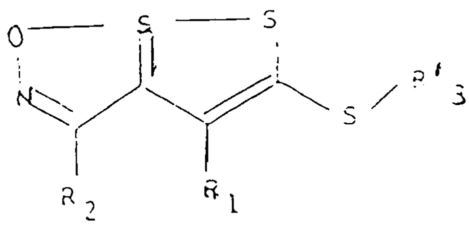

(VIII)

in der $R_1$ und $R_2$ die in Anspruch 1 angegebenen Bedeutungen besitzen und $R'_3$ ausgewählt ist aus einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe,
**dadurch gekennzeichnet**, daß man ein Oxim der Formel II:

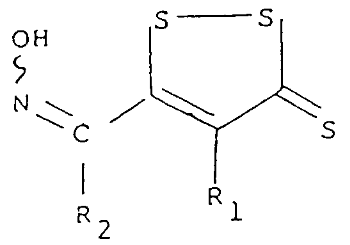

(II)

mit einem Halogenid der Formel

$R'_3$-Hal

worin $R'_3$ die oben angegebenen Bedeutungen besitzt, in Gegenwart einer wäßrigen Natriumhydroxidlösung alkyliert.

**14.** Verfahren zur Herstellung von Verbindungen der Formel

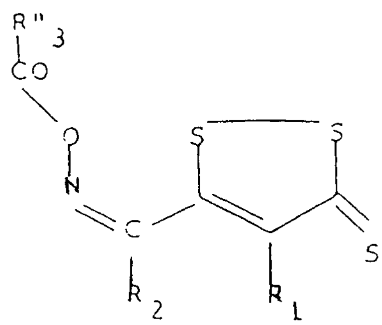

(IX)

in der $R_1$ und $R_2$ die in Anspruch 1 angegebenen Bedeutungen besitzen und $R''_3$ ausgewählt ist aus einem Wasserstoffatom, einer $C_1$-$C_6$-Alkylgruppe, einer $C_1$-$C_6$-Alkylgruppe, die durch eine oder mehrere Gruppen ausgewählt aus Hydroxylgruppen, Aminogruppen, Chloratomen und $C_1$-$C_4$-Alkoxygruppen substituiert ist, und einer Aryl-($C_1$-$C_6$-alkyl)-gruppe,
**dadurch gekennzeichnet,** daß man ein Oxim der Formel II

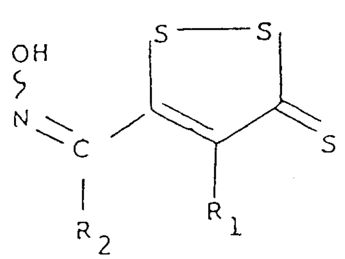

(II)

mit einem Säurechlorid der Formel

$R''_3COCl$

worin $R''_3$ die oben angegebenen Bedeutungen besitzt, in Gegenwart von Pyridin in Toluol acyliert.

**15.** Verfahren zur Herstellung der Verbindung der Formel

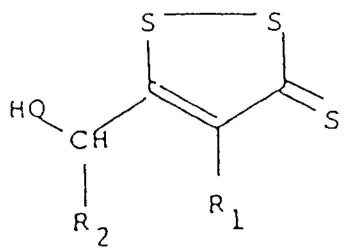

(X)

worin $R_1$ und $R_2$ die in Anspruch 1 angegebenen Bedeutungen besitzen, **dadurch gekennzeichnet**, daß man eine Verbindung der Formel VI

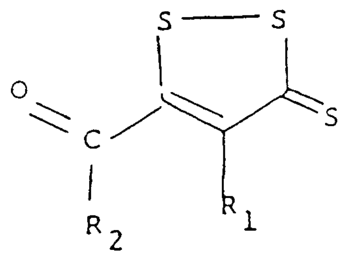

(VI)

reduziert.

**16.** Verfahren zur Herstellung von Verbindungen der Formel:

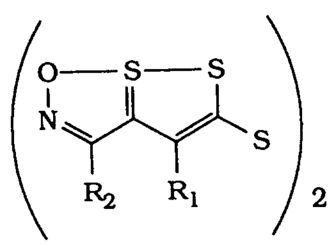

(V)

in der $R_1$ und $R_2$ die in Anspruch 1 angegebenen Bedeutungen besitzen, **dadurch gekennzeichnet,** daß man eine Verbindung der Formel

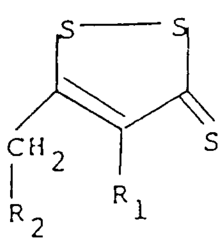

(III)

mit Natriumnitrit in Eisessigmedium behandelt.